# EUROPEAN PATENT SPECIFICATION

(11) **EP 1 709 168 B1**
(45) Date of publication and mention of the grant of the patent: **03.03.2010**
(21) Application number: 05706913.0
(22) Date of filing: 13.01.2005
(51) Int. Cl.: C12N 9/52, C12N 15/62, C12N 15/57, C07K 16/46, C12N 15/63, C12N 1/21, C12P 21/02

(54) **GENERATION OF SPECIFIC ADHESION IN GRAM-NEGATIVE BACTERIA BY MEANS OF ANCHORING IMMUNOGLOBULIN SINGLE DOMAINS ON THEIR SURFACE WITH AUTOTRANSPORTERS**
ERZEUGUNG SPEZIFISCHER ADHÄSION BEI GRAM- NEGATIVEN BAKTERIEN MITTELS VERANKERUNG VON EINZELDOMÄNEN AUS IMMUNGLOBULINEN AUF DEREN OBERFLÄCHE MIT AUTOTRANSPORTERN
PRODUCTION D'ADHERENCE SPECIFIQUE DANS DES BACTERIES GRAM NEGATIF PAR FIXATION DE DOMAINES UNIQUES D'IMMUNOGLOBULINES SUR LA SURFACE DESDITES BACTERIES AU MOYEN D'AUTOTRANSPORTEURS

(30) Priority: 14.01.2004 ES 200400073
(43) Date of publication of application: 11.10.2006
(73) Proprietor: CONSEJO SUPERIOR DE INVESTIGACIONES CIENTIFICAS, 28006 Madrid (ES)
(72) Inventor: VEIGA CHACON, Esteban, E-28006 Madrid (ES); DE LORENZO PRIETO, Victor, E-28006 Madrid (ES); FERNANDEZ HERRERO, Luis Angel, E-28006 Madrid (ES)
(74) Representative: ABG Patentes, S.L.
(86) International application number: PCT/EP2005/000444
(87) International publication number: WO 2005/068619

(56) References cited:
- VEIGA ESTEBAN ET AL: "Neutralization of enteric coronaviruses with Escherichia coli cells expressing single-chain Fv-autotransporter fusions." JOURNAL OF VIROLOGY, vol. 77, no. 24, December 2003 (2003-12), pages 13396-13398, XP002328574 ISSN: 0022-538X
- VEIGA ESTEBAN ET AL: "Autotransporters as scaffolds for novel bacterial adhesins: Surface properties of Escherichia coli cells displaying Jun/Fos dimerization domains." JOURNAL OF BACTERIOLOGY, vol. 185, no. 18, September 2003 (2003-09), pages 5585-5590, XP002328575 ISSN: 0021-9193
- VEIGA E ET AL: "PROBING SECRETION AND TRANSLOCATION OF A BETA-AUTOTRANSPORTER USINGA REPORTER SINGLE-CHAIN FV AS A COGNATE PASSENGER DOMAIN" MOLECULAR MICROBIOLOGY, BLACKWELL SCIENTIFIC, OXFORD, GB, vol. 33, no. 6, September 1999 (1999-09), pages 1232-1243, XP001007312 ISSN: 0950-382X cited in the application
- VEIGA ESTEBAN ET AL: "Structural tolerance of bacterial autotransporters for folded passenger protein domains" MOLECULAR MICROBIOLOGY, vol. 52, no. 4, May 2004 (2004-05), pages 1069-1080, XP002328576 ISSN: 0950-382X
- ELS CONRATH K ET AL: "Camel single-domain antibodies as modular building units in bispecific and bivalent antibody constructs" JOURNAL OF BIOLOGICAL CHEMISTRY, AMERICAN SOCIETY OF BIOLOGICAL CHEMISTS, BALTIMORE, MD, US, vol. 276, no. 10, 9 March 2001 (2001-03-09), pages 7346-7350, XP002248402 ISSN: 0021-9258

## Description

### FIELD OF THE INVENTION

The present invention refers to the use of genetic tools which allow the expression of different proteins on the surface of gram-negative bacteria. The present invention particularly refers to the use of autotransporters as carriers for the presentation of recombinant antibodies which specifically bind to known antigens and to thus redirect the bacterial adhesion.

### BACKGROUND OF THE INVENTION

The proteins located on the surface of the bacteria, or secreted to the extracellular medium, are necessary for a number of biological phenomenons, such as cell recognition, host cell adhesion and invasion, conjugation, bacteriophage assembly and cell mobility, inter alia (Buttner and Bonas, Trends Mircobiol 2002, 10, 186-192; Cabanes et al., Trends Microbiol 2002, 10, 238-245; Cao and Saier, Microbiology 2001, 147, 3201-3214; Christie, Mol Microbiol 2001, 40, 294-305; Fernández and Berenguer, FEMS Microbiol Rev 2000, 24, 21-44; Finlay and Falkow, Microbiology and Molecular Biology Reviews 1997, 61, 136-169; Giron et al., Mol Microbiol 2002, 44, 361-379; Hueck, Microbiol Mol Biol Rev 1998" 62, 379-433; Lee and Schneewind, Genes Dev 2001, 15, 1725-1752; Soto and Hultgren, J Bacteriol 1999, 181, 1059-1071).

Protein secretion is particularly complex in gram-negative bacteria, since they must pass through two lipid membranes. To carry out this task, gram-negative bacteria have developed an array of secretion systems classified in five large groups depending on the molecular nature of the transport machinery.

It is also possible to point out the existence of systems specialized in fimbria secretion (chaperone/usher). These systems are mechanistically divided into two large groups: those in which there is no periplasmic intermediary (sec-independent), and those with secretion with one stage in the periplasm (sec-dependent).

Autotransporters (ATs) were initially disclosed as a family of secreting proteins, all the information necessary for their translocation through the outer membrane (OM) resides in the polypeptide that is exported. The C-terminal region of the ATs acts as a secretion mechanism, translocating the N-terminal domain through the OM without the aid of any other known protein (Henderson et al., Trends Microbiol 2000, 8 (12): 529-532; Henderson et al., Trends Microbiol 1998, 6 (9): 370-8; Holland, Trends Microbiol 1998, 6 (10): 388-9; Pohlner et al., Nature 1987, 325 458-462). The ATs are a widely distributed family among gram-negative bacteria. To date, about 120 members belonging to 20 different genres of α-, β-, γ- and ε-proteobacteria and chlamydias have been identified (Yen et al., Biochim Biophys Acta 2002, 1562 (1-2): 6-31).

Some examples of ATs as virulence factors present in human pathogens are *Neisseria gonorrhoeae, N. meningitidis* and *Haemophilus influenzae* IgA proteases (Pohlner et al., Nature 1987, 325 458-462; Lomholt et al., Mol. Microbiol. 1995, 15 495-506), the polymerization factor of *Shigella flexnerii* IcsA actin (Suzuki et al., J. Biol. Chem. 1995, 270 30874-30880), AIDA-I adhesin of pathogenic *Escherichia coli* strains (Benz and Schmidt, Mol Microbiol 1992, 6, 1539-1546; Suhr et al., Mol. Microbiol. 1996, 22, 31-42), the factor of resistance to the *Bordetella pertussis* BrkA complement (Fernández and Weiss, Infect. Immun. 1994, 62 4727-4738), and the *Helicobacter pylori* VacA cytotoxin (Schmitt and Haas, Mol. Microbiol. 1994, 12 307-319).

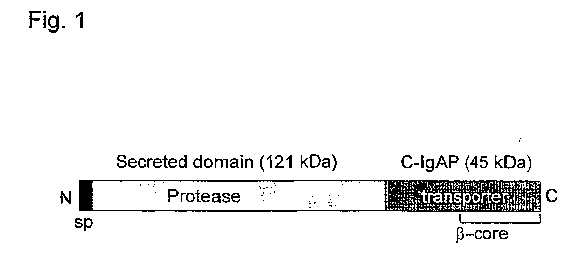

All the ATs have the same modular structure (see Figure 1). From the N-terminal end: **I**, a signal peptide directing the translocation of the pre-protein towards the periplasm through the inner membrane (IM) (Henderson et al., Trends Microbiol 1998, 6, 370-378; Pohlner et al., Nature, 1987, 325, 458-462; Sijbrandi et al., J Biol Chem 2002, 3, 3); **II**, a variably-sized passenger domain (from 0 to more than 250 kDa), which is translocated through the OM and is responsible for the biological extracellular role; **III**, a transporter domain (β) located in the C-terminal end (sometimes referred herein as C-terminal β-domain or C-terminal domain or transporter), conserved among the members of the family, and that directs the secretion through the OM of the passenger domain (Pohlner et al., Nature, 1987, 325, 458-462; Klauser et al., J. Mol. Biol. 1993, 234 579-593; Maurer et al., J Bacteriol 1999, 181 (22): 7014-20).

The passenger domain can be processed and released to the medium (*N. gonorrhoeae* IgA protease), it can be processed and remain associated to the OM (*E*. *coli* Ag43 adhesin) or it can go unprocessed and remain exposed towards the medium (*H*. *influenzae* Hia adhesin).

Several members of the family of the ATs have been used for the exposure of different polypeptides fused to the β domain thereof, such as rat metallothionein for example (Valls et al., Nat. Biotechnol. 2000, 18; 661-5), cholera toxin subunit B (Maurer et al., J. Bacteriol. 1997, 794-804), β-lactamase (Lattemann et al., J. Bacteriol. 2000, 182: 3726-33), bovine adrenodoxin (Jose et al., J. Biotechnol. 2002, 95: 257-68), carboxylesterase EstA from *Burkholderia gladioli* (J. Mol. Catal. B: Enzymatic 2002, 18: 89-97), the fimbrial protein FimH (Kjaergaard et al., J. Bacteriol. 2002, 184: 4197-204), inter alia.

The work aimed at the exposure of polypeptides on the bacterial surface by means of the use of different members of the family of ATs indicates the need to prevent systems with disulfide bonds (Jose, J. et al., 1996, 178 (107-110); (Klauser, T. et al., 1992, 11 (2327-2335)). This fact is also clearly shown in the work of Veiga et al. (Mol. Microbiol. 1999, 33(6): 1232-1243), which discloses the secretion and translocation as a passenger domain of a single chain Fv fragment (which has two intramolecular disulfide bonds stabilizing their tertiary structure and which are required for scFvs to be functional), this translocation is achieved with a low efficiency rate, having problems of proteolysis of the fusion, in addition to problems of toxicity for the bacteria.

The miniantibodies or recombinant antibodies are fragments derived from the antibodies built by recombinant DNA technology, and in spite of their smaller size, they conserve the antigen binding capacity given that they maintain the variable domains (V) of an immunoglobulin (Ig), where the antigen binding areas are located.

One type of miniantibodies are those referred to as scFv (Plückthun, et al., Antibody Engineering: A Practical Approach 1996), 203-252). These scFv are chimeric proteins of about 30 kDa resulting from the fusion in the same polypeptide chain of the variable domains (V_{H} and V_{L}) of the heavy (H) and light (L) chains of an antibody through a flexible peptide (usually 20 amino acid repetitions of glycine and serine) allowing the quaternary interactions between both Ig domains to be conserved. The binding capacity of these scFv resides in the surface generated by the interaction of the two variable domains. These scFv have two intramolecular disulfide bonds stabilizing their tertiary structure and required for the scFv to be functional.

Other miniantibodies successfully expressed in *E*. *coli* are those called V_{HH} (Nguyen et al., Adv Immunol 2001, 79 261-296; Nguyen et al., Embo J 2000, 19 (5): 921-30; Sheriff and Constantine, Nat Struct Biol 1996, 3 (9): 733-6). In camelidae species (camels, dromedaries, Ilamas, etc.), part of their antibodies is light-chain deficient (Hamers-Casterman et al., Nature 1993, 363 (6428): 446-8; Muyldermans et al., Protein Eng 1994, 7 (9): 1129-35), the recognition area of the antigens being constituted only of the V_{H} domain (Desmyter et al., J Biol Chem 2001, 276 (28): 26285-90; Desmyter et al., J Biol Chem 2002, 277 (26): 23645-50; Desmyter et al., Nat Struct Biol 1996, 3 (9): 803-11). This V_{H} domain differs from the V_{H} domains of other antibodies in the sense that its antigen recognition areas are formed by larger loops. Thus, the camelidae antibody V_{H} domains are called V_{HH}. This about 15 kDa domain (V_{HH}) can be expressed in the *E*. *coli* periplasm maintaining its antigen binding capacity. The V_{HH} are more stable molecules than the scFv, and unlike the latter, they rarely aggregate (J Immunol. Methods 1999, 231: 25-38).

Just as V_{L} and V_{H} domains in mice, V_{HH} domains have an intramolecular S-S bond stabilizing their tertiary structure and necessary for their proper folding (Desmyter et al., J Biol Chem 2002, 277 (26): 23645-50; Desmyter et al., Nat Struct Biol 1996, 3 (9): 803-11).

Veiga et al. (Mol Microbiol 1999, 33: 1232-1243) disclosed the secretion of an scFv miniantibody (which has two intramolecular disulfide bonds stabilizing its tertiary structure) as a passenger fused to the C-terminal domain of *N*. *gonorrhoeae* IgA protease (C-IgAP) with an efficiency of about 20%, further having problems of proteolysis of the fusion and of toxicity for the bacteria.

### SUMMARY OF THE INVENTION

According to the present invention, the anchoring and expression of recombinant antibodies with a single immunoglobulin domain (single-domain) on the surface of the outer membrane (OM) of a bacteria, i.e., on the external surface of the bacterial OM, has been unexpectedly achieved by using a transporter domain of an autotransporter (AT) with a notably higher efficiency than that expected, and decreasing the problems both of proteolysis of the fusion and toxicity for the bacteria, in spite of the fact that said single-domain antibodies have disulfide bonds in their tertiary structure.

Therefore, in an aspect, the invention relates to a gene construction comprising (i) a nucleotide sequence encoding a signal peptide, (ii) a nucleotide sequence encoding a single-domain recombinant antibody; and (iii) a nucleotide sequence encoding the C-terminal domain of an AT; wherein the 3' end of said sequence (i) is linked to the 5' end of said sequence (ii) and the 3' end of said sequence (ii) is linked to the 5' end of said sequence (iii).

In other aspect, the invention relates to an expression vector comprising said gene construction operatively linked to a transcription control sequence. In an embodiment said transcription control sequence is functional in bacteria.

An expression vector for single-domain recombinant antibodies on the surface of the bacterial OM, characterized in that the expressed antibody is secreted by a transporter domain of an AT, constitutes an additional aspect of this invention.

In other aspect, the invention relates to gram-negative bacteria comprising said gene construction or said expression vector.

In other aspect, the invention relates to a hybrid protein obtainable by the expression of nucleic acid sequence contained in said gene construction or in said expression vector.

In other aspect, the invention relates to a method of anchoring and expressing a single-domain recombinant antibody on the surface of the OM of a bacteria which comprises culturing a bacteria containing said gene construction or said expression vector under conditions which allow for the production of said single-domain recombinant antibody, its anchoring and expression on the surface of the OM of said bacteria in the form of a hybrid protein.

In other aspect, the invention relates to a method for the specific adhesion of a bacteria to an antigen, wherein said bacteria has a single-domain recombinant antibody capable of recognizing (binding to) said antigen anchored on the surface of its OM.

In other aspect, the invention relates to a method for producing a single-domain recombinant antibody anchored on the surface of the OM of a bacteria.

### BRIEF DESCRIPTION OF THE DRAWINGS

Figure 1 shows a representation of the modular organization of the ATs. The N and C terminal ends are indicated, as well as the signal peptide (sp), the translocated passenger domain and the β domain.

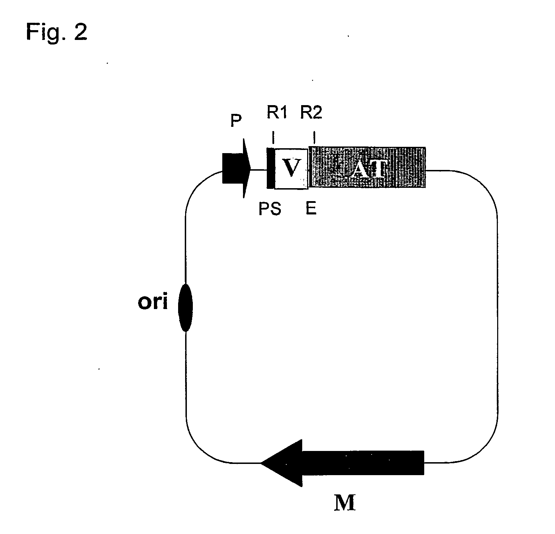

Figure 2 shows a representation of the basic structure of a expression vector of the fusions between immunoglobulin V domains and transporter domains. The encoding DNA of the V domain is between encoding sequences of a signal peptide (SP) and the autotransporter domain (AT). The V domain is ideally flanked by two single restriction sites (R1, R2) and the V-AT fusion contains an epitope (E) for detection thereof by means of a specific antibody. The vector further contains the following elements: a replication origin (ori), a marker gene (M) for its selection, and promoter sequences (P) for the transcription of the V-AT fusion.

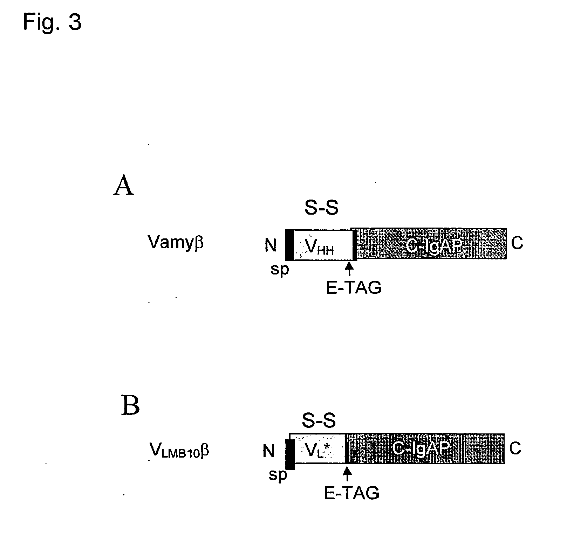

Figure 3 shows: (A) A representation of the modular organization of V_{amy}β, wherein the N and C terminal ends are indicated, as well as the signal peptide (sp), the translocated passenger domain V_{amy} (represented as V_{HH} in the figure) and the C-terminal β-domain of *N. gonorrhoeae* IgA protease (C-IgAP). (B) A representation of the modular organization of V_{LMB10}β, wherein the N and C terminal ends are indicated, as well as the signal peptide (sp), the translocated passenger domain V_{LMB10} (represented as V_{L}* in the figure) and the C-terminal β-domain of *N. gonorrhoeae* IgA protease (C-IgAP). Both proteins include an epitope (E-TAG) for its detection by a specific antibody.

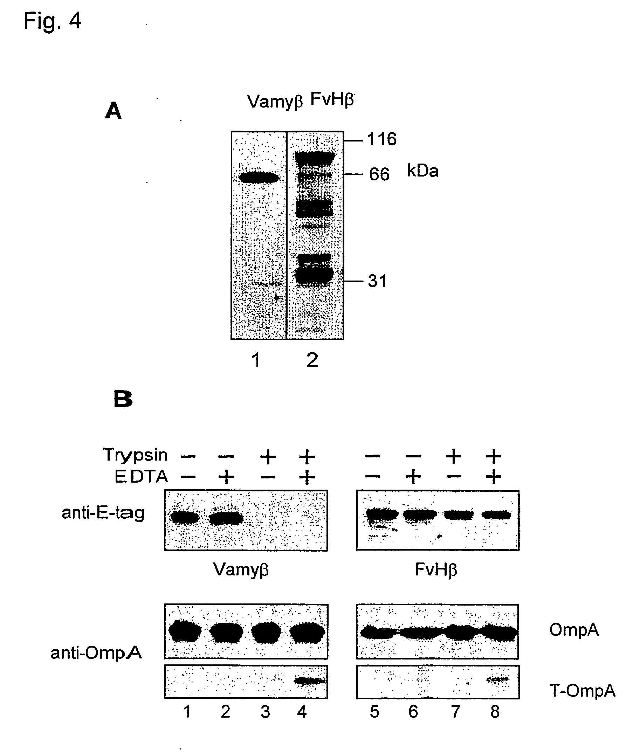

Figure 4 shows: (A) Immunodetection by Western-blot using the anti-E-tag monoclonal antibody (mAb) of the different hybrid proteins (V_{amy}β and FvHβ) from total *E. coli* UT5600 cell extracts. In the case of V_{amy}β, a single band corresponding to 66 kDa is observed, whereas in the case of FvHβ, several bands corresponding to the proteolysis of the fusion are observed. (B) Exposure of the V_{amy}β and FvHβ hybrid proteins on the surface of *E. coli* UT5600. When (+) is indicated, the cells were permeabilized with 10 mM EDTA (ethylendiaminetetraacetic acid), or they were incubated with trypsin (1 µm/ml) added externally. The presence of the different hybrid proteins in the crude extracts was determined by Western-blot with the anti-E-tag mAb. The amount of OmpA in the same extracts (determined using a polyclonal rabbit anti-OmpA serum) is shown as a loading control. To detect the about 24 kDa fragment (T-OmpA) corresponding to the tryptic degradation of the periplasmic domain of OmpA, the photographic films were overexposed.

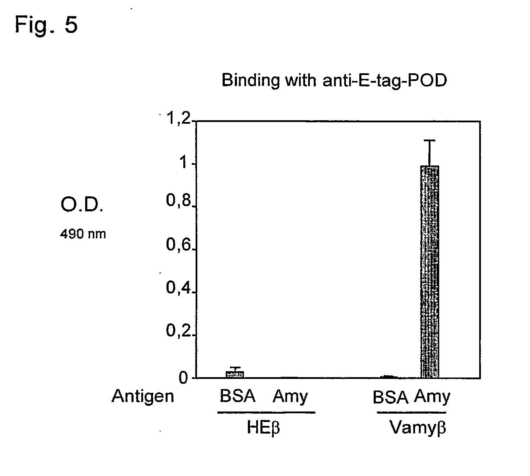

Figure 5 shows a functionality/folding assay of the passenger domains of the different constructs derived from C-IgAP exposed towards the extracellular medium. ELISA plates were coated with α-amylase (Amy) and with bovine serum albumin (BSA) as an antigen control. The *E. coli* UT5600 cells which expressed these hybrid proteins were added on the ELISA plates and the binding of the passengers exposed on the surface of these cells was detected with the anti-E-tag mAb linked to peroxidase (POD). The *E. coli* cells which expressed the HEβ protein were used as a negative control.

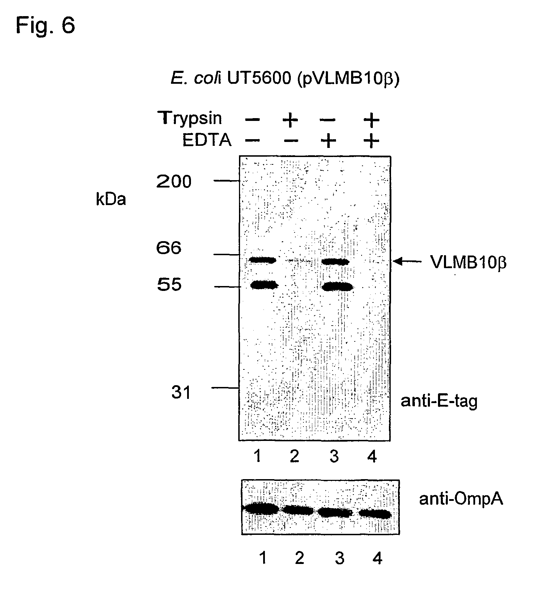

Figure 6 shows the exposure of the V_{LMB10}β hybrid on the surface of *E. coli* UT5600. When (+) is indicated, the cells were permeabilized with 10 mM EDTA, or they were incubated with trypsin (1 µg/ml) added externally. The presence of the different hybrid proteins in the crude extracts was measured by Western-blot with the anti-E-tag mAb. The amount of OmpA in the same extracts (measured using a polyclonal rabbit anti-OmpA serum) is shown as a loading control. An exposure of over 90% of the fusion protein (65 kDa band) is observed, there also being a single band of proteolytic degradation of about 55 kDa.

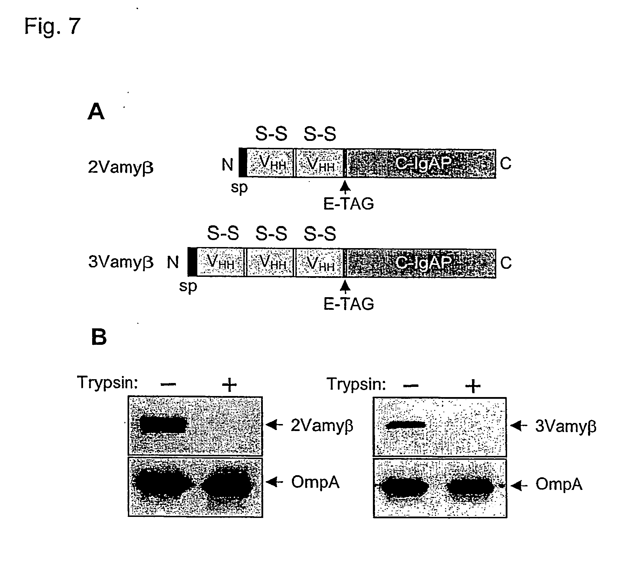

Figure 7 shows: (A) The modular organization of 2V_{amy}β and 3v_{amy}β. (B) The exposure of the 2V_{amy}β and 3V_{amy}β hybrid proteins on the surface of *E. coli* UT5600. When (+) is indicated, the cells are incubated with trypsin (1 µg/ml) added externally. The presence of the different hybrid proteins in the crude extracts was measured by Western-blot with the anti-E-tag mAb. The amount of OmpA in the same extracts (measured using a polyclonal rabbit anti-OmpA serum) is shown as a loading control.

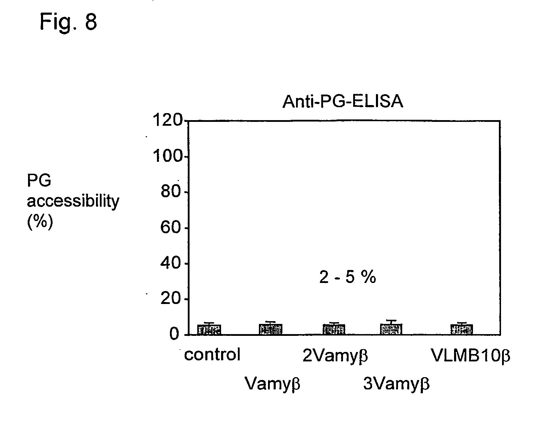

Figure 8 shows the peptidoglycan (PG) accessibility assay in which the control (pAKnot), V_{amy}β, 2V_{amy}β, 3V_{amy}β and V_{LMB10}β constructs were included. In all of them, only one PG accessibility level occurs at about 5% maximum and identical to that observed in the control strain which does not express any hybrid protein.

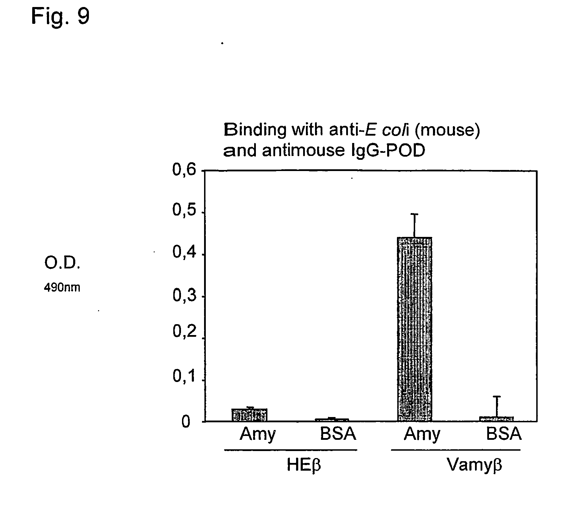

Figure 9 shows the specific adhesion of *E. coli* cells to a surface containing an amylase antigen (Amy) or control antigen (BSA). The binding of the *E. coli* cells to the plates was detected with a polyclonal anti-*E*. *coli* serum generated in mice. The *E. coli* cells which expressed the HEβ protein were used as a negative control.

### DETAILED DESCRIPTION OF THE INVENTION

The invention relates, in general, to the anchoring and expression of single-domain recombinant antibodies on the bacterial surface, secreted by a transporter domain of an AT. Thus, the invention provides an expression vector for single-domain recombinant antibodies on the surface of the bacterial OM, characterized in that the expressed antibody is secreted by a transporter domain of an AT.

Therefore, in an aspect, the invention relates to a gene construction, hereinafter referred to as the gene construction of the invention, comprising:
a) a first nucleic acid sequence containing the nucleotide sequence encoding a signal peptide;
b) a second nucleic acid sequence containing the nucleotide sequence encoding a single-domain recombinant antibody; and
c) a third nucleic acid sequence containing the nucleotide sequence encoding the C-terminal domain of an AT;
wherein the 3' end of said first nucleic acid sequence is linked to the 5' end of said second nucleic acid sequence and the 3' end of said second nucleic acid sequence is linked to the 5' end of said third nucleic acid sequence.

The first nucleic acid sequence comprises the nucleotide sequence encoding a signal peptide. A signal peptide is a peptide sequence, usually present in the N-terminal end of may secretory proteins (i.e., which are secreted or exported from the place they are produced) or membrane proteins, involved in the passage of the protein across the cell membrane (in bacteria). Practically any signal peptide may be used in the instant invention. Illustrative, non limitative, examples of said signal peptide directing the passage of the hybrid protein provided by the instant invention towards the periplasm include the signal peptide of PelB (Keen, N.T. et al., J Bacteriol 1986, 168 (2) 595-606), the signal peptide of OmpA (Movva, N.R. et al., J Biol Chem 1980, 255 (1) 27-9), the signal peptide of protein 3 of bacteriophage M13 (Parmley, S.F. et al., Adv Exp Med Biol 1989, 251 (215-8)), the signal peptide of the maltose binding protein (MBP) (Bedouelle, H. et al., Eur J Biochem 1988, 171 (3) 541-9), or any other signal peptide of those normally used with AT systems. In a particular embodiment, said signal peptide is the signal peptide of PelB.

The second nucleic acid sequence comprises the nucleotide sequence encoding a single-domain recombinant antibody. Within the framework of the present invention, a "single-domain recombinant antibody" refers to an immunoglobulin-type domain with independent binding and recognition capacity, such as a natural or modified heavy chain variable domain (V_{H}) of an antibody, a natural or modified light chain variable domain (V_{L}) of an antibody, a natural or modified recombinant camelide antibody (V_{HH}), a humanized recombinant camelide antibody, a recombinant antibody of a non-camelide animal engineered in order to make it capable of interacting in the form of a single-domain with its antigen (i.e., "camelized"), an IgNAR single-domain antibody of cartilaginous fish, etc. The gene construction may contain a combination of nucleotide sequences encoding single-domain recombinant antibodies- The gene construction of the invention may contain a nucleotide sequence encoding one single-domain recombinant antibody or the nucleotide sequences encoding two or more, equal. In the last case, said single-domain recombinant antibodies may be, optionally, separated from each other by some spacers (i.e., peptide sequences which allow for separating said single-domain recombinant antibodies each other, which will be described later in a detailed manner). In a particular embodiment, the gene construction of the invention comprises a nucleotide sequence encoding just one single-domain recombinant antibody. In another particular embodiment, the gene construction of the invention comprises the nucleotide sequences encoding two or more, for example, three, equal single-domain recombinant antibodies. Said single-domain recombinant antibody or antibodies constitute, therefore, the passenger domain or domains to be secreted by the AT system present in the gene construction of the invention or in the expression vectors provided by this invention.

As used herein, the term "natural", applied to a product, refers to the product as found in nature, without having been subjected to human manipulation, whereas the term "modified" refers to any change carried out by the human being on a natural product, e.g., changes introduced in natural products in order to modify or improve their stability and/or solubility properties. Also, the term "non-camelide animal" refers to an animal capable of generating immunoglobulins which does not pertain to the camelidae family [family of mammals of the artiodactyls order, tilopodes suborder]. Therefore, the term "single-domain recombinant antibody", as used herein, includes both the antigen recognition regions of antibodies which are naturally single-domain (e.g., V_{HH} and IgNAR), and antigen recognition regions of antibodies which, by genetic engineering, have been altered so that they are able to interact with the antigen on their own and improve their properties of stability and/or solubility.

Although practically any single-domain antibody capable of being recombinantly expressed may be used in the instant invention, in a particular embodiment, with illustrative, non limitative, purposes, of the invention, a V_{HH} recognizing α-amylase (V_{amy}) as representative of a natural single-domain recombinant antibody was used (Example 1). Said V_{amy} contains a single Ig domain, its size being the half (about 15 kDa) of the size corresponding to an scFv (about 30 kDa). As representative of a genetically engineered single-domain recombinant antibody so that it would be able to interact with the antigen on its own and improve its stability and solubility properties, a V_{L} of human origin (MB10 clone) against the B7.1 cell surface marker (van den Beucken et al., J. Mol. Biol. (2001) 310, 591-601) was used (Example 2).

The third nucleic acid sequence comprises the nucleotide sequence encoding the C-terminal domain of an AT. The C-terminal domain (transporter) of an AT acts as a secretion machinery translocating the N-terminal end through the OM. Practically, for carrying out the invention it may be used the C-terminal domain of any AT capable of translocating the passenger domain (single-domain recombinant antibody or antibodies) to which it is bound, across the OM of a bacteria, its anchoring and exposure on the external surface of the OM of said bacteria. In a particular embodiment, said third nucleic acid sequence comprises the nucleotide sequence encoding the C-terminal domain of an AT of a gram-negative bacteria. Illustrative, non limitative, examples of said ATs include IgA proteases of *Neisseria spp.* or *Hemophilus spp.,* for example, *N. gonorrhoeae, N. meningitides, H. influenzae* IgA proteases (Pohlner et al., Nature 1987, 325 458-462; Lomholt et al., Mol. Microbiol. 1995, 15 495-506), the polymerization factor of *Shigella flexnerii* IcsA actin (Suzuki et al., J. Biol. Chem. 1995, 270 30874-30880), AIDA-I adhesin of pathogenic *Escherichia coli* strains (Benz and Schmidt, Mol Microbiol 1992, 6, 1539-1546; Suhr et al., Mol. Microbiol. 1996, 22, 31-42), the factor of resistance to the *Bordetella pertussis* BrkA complement (Fernández and Weiss, Infect. Immun. 1994, 62 4727-4738), the *Helicobacter pylori* VacA cytotoxin (Schmitt and Haas, Mol. Microbiol. 1994, 12 307-319), etc. In a specific embodiment, said third nucleic acid sequence comprises the nucleotide sequence encoding the C-terminal β-domain of *N. gonorrhoeae* IgA protease. Information concerning sequences of AT is available to the public and may be obtained, for example, from http://www.sanger.ac.uk/cgi-bin/Pfam/getacc?PF03797.

The expression of the nucleic acid sequence contained in the gene construction of the invention in a suitable expression system results in a hybrid protein (fusion protein) which comprises a domain (A) comprising the amino acid sequence of, at least, a single-domain recombinant antibody, and a domain (B) comprising the amino acid sequence of the C-terminal domain of an AT.

Generally, said domain (A) is not directly fused to said domain (B) since it may be advantageous to introduce between them a spacer (peptide sequence which allows for separating said domains (A) and (B) from each other). Therefore, if desired, the gene construction of the invention may contain, in addition, a fourth nucleic acid sequence encoding a spacer placed between said second and third nucleic acid sequences, wherein the 5' end of said fourth nucleic acid sequence is linked to the 3' end of said second nucleic acid sequence and the 3' end of said fourth nucleic acid sequence is linked to the 5' end of said third nucleic acid sequence. Thus, the nucleotide sequence encoding the single-domain recombinant antibody or antibodies is joined to the nucleotide sequence encoding the C-terminal domain of an AT by means of a nucleotide sequence encoding a spacer. Advantageously, said spacer is a peptide sequence having structural flexibility (i.e., flexible). Practically any peptide sequence having structural flexibility may be used. Said flexible peptide sequence may comprise, for example, multimers or repetitions of amino acid residues, such as, alanine (A), glycine (G), etc., the peptide sequence -AAAAGA- or any other suitable non repetitive sequence of amino acid residues such as, for example, the non repetitive spacer of sequence - TPSHNSHQVPSAGGPTANSG- (one-letter code of amino acids), etc., or the hinge region of an antibody.

When the hybrid protein provided by the instant invention as a result of the expression of the nucleic acid sequence contained in the gene construction of the invention comprises two or more equal single-domain recombinant antibodies, said antibodies may be, optionally, separated from each other by spacers of the previously defined type, so that said single-domain recombinant antibodies are not directly fused between them but through said spacers. Therefore, if desired, the gene construction of the invention may further contain a nucleic acid sequence encoding a spacer placed between two nucleic acid sequences encoding said single-domain recombinant antibodies. Thus, the nucleotide sequences encoding the single-domain recombinant antibodies are joined to each other by a nucleotide sequence encoding a spacer. The same spacer used for separating the domains (A) and (B), previously defined, may be used for separating the single-domain recombinant antibodies from each other. In a particular embodiment, the spacer used is the non repetitive spacer of sequence -TPSHNSHQVPSAGGPTANSG- (one-letter code of amino acids) (Example 1, A48 constructions and V_{amy} repetitions).

In order to make easier the detection of the hybrid protein provided by the instant invention as a result of the expression of the nucleic acid sequence contained in the gene construction of the invention, said gene construction may contain, if desired, a nucleic acid sequence encoding a peptide sequence susceptible of being used for detection or recognition purposes of the hybrid protein of the invention. Therefore, in a particular embodiment, the gene construction of the invention comprises, if desired, a fifth nucleic acid sequence encoding a peptide sequence for detection purposes. Practically any peptide sequence which allows for detecting the hybrid protein of the invention may be used, for example, a peptide sequence susceptible of being recognized by an antibody (e.g., a monoclonal antibody) which can be used for recognizing or detecting the hybrid protein of the invention by conventional analytical methods (e.g., immunoaffinity chromatography, etc.), for example, a poly-hystidine sequence, the sequences of epitopes E, HA, FLAG, c-myc, etc. [Using Antibodies: A laboratory manual. Ed Harlow and David Lane (1999). Cold Spring Harbor Laboratory Press. New Cork. Capítulo: Tagging proteins. pp. 347-377] and, in general, any other sequence capable of being recognized by an antibody. In a particular embodiment, said peptide sequence susceptible of being used with detection purposes comprises the sequence of epitope E (E-tag).

The above mentioned fifth nucleic acid sequence is placed, advantageously, between said second and third nucleic acid sequences, wherein the 5' end of said fifth nucleic acid sequence is linked to the 3' end of said second nucleic acid sequence and the 3' end of said fifth nucleic acid sequence is linked to the 5' end of said third nucleic acid sequence. Nevertheless, said fifth nucleic acid sequence could be placed in any other position provided that the presence of said sequence guarantees the detection of the hybrid protein of the invention and does not affect the functionality of said hybrid protein.

The gene construction of the invention may be obtained by using techniques broadly known by the skilled person in the art [Sambrook et al., "Molecular cloning, a Laboratory Mariual", 2nd ed., Cold Spring Harbor Laboratory Press, N.Y., 1989 Vol 1-3]. Said gene construction of the invention may be used for the construction of vectors, such as expression vectors, specially useful for producing in gram-negative bacteria hybrid proteins comprising, at least, a single-domain recombinant antibody and the C-terminal domain of an AT.

Therefore, in other aspect, the invention relates to a vector, hereinafter referred to as vector of the invention, which comprises the gene construction of the invention. In a particular embodiment, the vector of the invention is an expression vector which comprises a gene construction of the invention operatively linked to a transcription control sequence. Consequently, the invention provides an expression vector for single-domain recombinant antibodies on the surface of the bacterial OM, characterized in that the expressed antibody is secreted by a transporter domain of an AT.

As used herein, "transcription control sequence" refers to a sequence which controls and regulates the transcription of the coding sequences contained in the gene construction of the invention. Said transcription control sequence comprises a promoter, a sequence encoding transcriptional regulators, a ribosome binding sequence (RBS) and/or a transcription terminator sequence. In a particular embodiment, said transcription control sequence is functional in bacteria, e.g., in gram-negative bacteria, and comprises an inducible or constitutive, transcription promoter functional in bacteria. Illustrative, non limitative, examples of inducible promoters include promoter pTetA which is induced by anhydrotetracycline (Skerra A., Gene 1994, 151 131-135); promoter pBAD which is induced by arabinose (Guzman L.M. et al., J Bacteriol 1995, 177(14) 4121-4130), the promoter of *Escherichia coli lac* operon (pLac) or its derivative promoter pTac, both induced by IPTG, lactose and other lactose analogues (Miller, The Operon 1978), promoter Pm which is induced by 3-methyl-benzoate and other benzoate analogues (Ramos, J.L. et al., Annu Rev Microbiol 1997,51 (3) 341-73), etc. Illustrative, non limitative, examples of constitutive promoters include the *E*. *coli* β-lactamase promoter (Klotsky, R.A. et al., Gene 1987, 55 (1) 141-6). In a particular embodiment, the induction of the expression of the expression vectors provided by this invention is under the control of promoter pLac and said induction may be obtained by adding IPTG, lactose or other lactose analog to the culture medium.

If desired, the vector of the invention may comprise, further, a marker, such as, for example, a gene which encodes a motif or phenotype which permits the selection of the host cell transformed with said vector. Illustrative, non limitative examples of said markers which could be present in the vector of the invention, such as in an expression vector provided by this invention, include genes of antibiotics resistance, e.g., genes of resistance to amplicillin, tetracycline, kanamycin, chloramphenicol, spectinomicin, etc.

The vector of the invention, once introduced into the host cell, may be integrated (or not) into the genome of said cell.

The vector of the invention may be obtained by using conventional techniques known by the skilled person in the art [Sambrook et al., "Molecular cloning, a Laboratory Manual", 2nd ed., Cold Spring Harbor Laboratory Press, N.Y., 1989 Vol 1-3].

In a particular embodiment, the invention provides an expression vector for gram-negative bacteria which allows for producing hybrid proteins, said hybrid proteins comprising, at least, a single-domain recombinant antibody and the C-terminal domain of an AT.

According to another preferred embodiment of the present invention, an expression vector for gram-negative bacteria is provided which allows for the production of hybrid proteins comprising, at least, a single-domain recombinant antibody and the C-terminal β-domain of *N*. *gonorrhoeae* IgA protease.

According to another particular embodiment of the present invention, the structure of the expression vectors derived from the *N*. *gonorrhoeae* IgA protease comprises, starting from the N-terminal end, an inducible or constitutive bacterial transcription promoter, the sequence encoding a signal peptide (which is the one that directs the passage of the hybrid proteins towards the periplasm), the sequence encoding, at least, a single-domain recombinant antibody (passenger domain) and the sequence encoding the C-terminal β-domain of *N*. *gonorrhoeae* IgA protease (which directs the secretion of the passenger domain towards the extracellular medium). Figure 2 depicts the structure of these vectors. Examples 1 and 2 describe the construction of some expression vectors derived from *N*. *gonorrhoeae* IgA protease comprising 1, 2 or 3 times the sequence encoding V_{amy} (V_{HH} which recognizes α-amylase) (Example 1) and the sequence encoding V_{LMB10}β (V_{L} of human origin (MB10 clone) against the B7.1 cell surface marker) (Example 2), together with the encoding sequence of the C-terminal β-domain of *N*. *gonorrhoeae* IgA protease (C-IgAp).

In other aspect, the invention relates to a bacteria, in particular, a gram-negative bacteria, comprising a gene construction of the invention or an expression vector of the invention, hereinafter referred to as bacteria of invention. Practically any gram-negative bacteria, for example, a *Escherichia spp.* strain (e.g., *E*. *coli*), etc., a *Salmonella spp.* strain (e.g., *S*. *tiphymurium,* etc.) and a *Pseudomonas spp.* strain (*P. aeruginosa, P. putida,* etc.), may be transformed with a gene construction of the invention or with a vector of the invention such as an expression vector provided by this invention. To that end, the promoter, regulatory and marker signals, as well as the replication origin will be optimized for each bacteria. In a particular embodiment, said gram-negative bacteria is a *E*. *coli* strain.

The gene construction of the invention as well as the expression vector of the invention may be used for transforming bacteria, in particular, gram-negative bacteria, thus generating transforming bacteria which carry, anchored on the external surface of its OM, at least, a single-domain recombinant antibody, in the form of a hybrid protein fused to the C-terminal domain of an AT; said bacteria being useful, thus, as systems for the presentation of antibodies, and may be used in a number of applications, including, among other:
- applications related to the bacterial adhesion for, for example, chelating metals, neutralizing pathogens (e.g., viral, etc.), specific adhesion to certain plants, bacterial aggregation, etc.;
- applications related to diagnostic purposes, in which case the single-domain recombinant antibody or antibodies present on the external surface of the OM of said bacteria will be an antibody capable of recognizing, or binding to, an antigen, such as a microbial antigen, i.e., an antigen of a microorganism including, without restricting to, viruses, bacteria, fungi and infectious parasites; a tumor antigen, i.e., a compound associated with a tumor or cancer ("tumor marker"), e.g., Her2 (breast cancer); GD2 (neuroblastome); EGF-R (malignant glioblastome); CEA (medular tyroid cancer); CD52 (leukemia); gp100 protein of human melanome; melan-A/MART-1 protein of human melanome; tyrosinase; NA17-A nt protein; MAGE-3 protein; p53 protein; HPV16E7 protein; etc., an auto-antigen, i.e., a protein encoded by the DNA of a subject and products generated by proteins or by RNA encoded by the DNA of a subject (WO 02/56905); and
- applications related to the selection of antibodies from antibodies collections by "bacterial display".

In other aspect, the invention relates to a hybrid protein, hereinafter referred to as hybrid protein of the invention, obtainable by the expression of the nucleic acid sequence contained in a gene construction according of the invention or in an expression vector provided by this invention.

In an embodiment, the hybrid protein of the invention comprises a domain (A) which comprises the amino acid sequence of, at least, a single-domain recombinant antibody, and a domain (B) comprising the amino acid sequence of the C-terminal domain of an AT. In a particular embodiment, said domain (B) comprises the amino acid sequence of the C-terminal β-domain of *N*. *gonorrhoeae* IgA protease.

The hybrid protein of the invention may also comprise a spacer between said domains (A) and (B) and/or a peptide sequence susceptible of being used for detection purposes, such as a peptide sequence susceptible of being recognized by an antibody, such as a poly-hystidine sequence, the sequences of epitopes E, HA, FLAG, c-myc, etc.

In a particular embodiment, hybrid proteins anchored to the external surface of the OM of *E*. *coli* comprising 1, 2 or 3 V_{amy} domains fused to the C-IgAp domain, referred to as V_{amy}β, V_{amy}2β and V_{amy}3β respectively (Figures 3A and 7A) (Example 1), have been obtained. In another particular embodiment, a hybrid protein anchored to the external surface of the OM of *E*. *coli* comprising the V_{LMB10} domain fused to the C-IgAp domain, hereinafter referred to as V_{LMB10}β (Figure 3B) (Example 2). In all the cases, the signal peptide of PelB directs the secretion of said hybrid proteins through the IM to the periplasmic space.

In other aspect, a method of anchoring and expressing a single-domain recombinant antibody on the surface of the OM of a bacteria which comprises culturing a bacteria of the invention under conditions which allow for the production of said single-domain recombinant antibody, its anchoring and expression on the surface of the OM of said bacteria (i.e., in the external surface of said bacterial OM) in the form of a hybrid protein fused to the C-terminal domain of an AT, such as the C-terminal domain of a gram-negative bacteria, e.g., the C-terminal β-domain of *N. gonorrhoeae* IgA protease. In a particular embodiment, said bacteria is a gram-negative bacteria provided by this invention, such as a *Escherichia spp.* strain (e.g., *E*. *coli*), etc., a *Salmonella spp.* strain (e.g., *S*. *tiphymurium,* etc.), a *Pseudomonas spp.* strain (P. *aeruginosa, P. putida,* etc.), etc. Conditions for optimizing the culture of the bacteria of the invention will depend on the bacteria used.

In other aspect, the invention relates to a method for the specific adhesion of a bacteria to an antigen which comprises the steps of:
a) transforming a bacteria with a gene construction of the invention or with an expression vector, said gene construction or expression vector comprising the nucleotide sequence encoding a single-domain recombinant antibody capable of recognizing said antigen;
b) culturing said transformed bacteria under conditions which allow for the production of said single-domain recombinant antibody, its anchoring and expression on the surface of the OM of said bacteria; and
c) bringing together the transformed bacteria and cultured from step b) with said antigen.

When culturing and growing the transformed bacteria under conditions which allow for the production of said single-domain recombinant antibody [step b)], due to the particulars of the gene construction or vector used, takes place its anchoring and expression on the surface of the OM of said bacteria (i.e., on the external surface of said bacterial OM) of said single-domain recombinant antibody in the form of a hybrid protein fused to the C-terminal domain of an AT, such as the C-terminal domain of a gram-negative bacteria, e.g., the C-terminal β-domain of *N*. *gonorrhoeae* IgA protease. In a particular embodiment, said bacteria is a gram-negative bacteria provided by this invention, such as a *Escherichia spp.* strain (e.g., *E*. *coli*), etc., a *Salmonella spp.* strain (e.g., *S*. *tiphymurium,* etc.), a *Pseudomonas spp.* strain (*P*. *aeruginosa, P. putida,* etc.), etc. Conditions for optimizing the culture of the bacteria of the invention will depend on the bacteria used.

In other aspect, the invention relates to a method for producing a single-domain recombinant antibody anchored on the surface of the OM of a bacteria which comprises culturing a bacteria of the invention under conditions which allow for the production of said single-domain recombinant antibody, its anchoring and expression on the surface of the OM of said bacteria (i.e., in the external surface of said bacterial OM) in the form of a hybrid protein fused to the C-terminal domain of an AT, such as the C-terminal domain of a gram-negative bacteria, e.g., the C-terminal β-domain of *N*. *gonorrhoeae* IgA protease. In a particular embodiment, said bacteria is a gram-negative bacteria provided by this invention, such as a *Escherichia spp.* strain (e.g., *E. coli*), etc., a *Salmonella spp.* strain (e.g., *S*. *tiphymurium,* etc.), a *Pseudomonas spp.* strain (*P. aeruginosa, P. putida,* etc.), etc. Conditions for optimizing the culture of the bacteria of the invention will depend on the bacteria used.

The present invention is illustrated by means of the following, non-limiting, examples.

### EXAMPLE 1

### Construction of hybrid proteins made up of the V_{HH} domain fused to C-IgAP

Hybrid proteins anchored to the external surface of the OM of *E*. *coli* comprising 1, 2 or 3 V_{amy} domains fused to the C-IgAp domain, hereinafter referred to as V_{amy}β, V_{amy}2β and V_{amy}3β respectively (Figures 3A and 7A), have been obtained. The signal peptide of PelB drives the secretion of said hybrid proteins through the IM to the periplasmic space.

### 1.1 Construction of the C-IgAP and V_{amy} hybrid protein (V_{amy}β)

An approximately 0.4 kb DNA fragment encoding a V_{HH} domain recognizing α-amylase (V_{amy}) was amplified by PCR using the phagemid A100R3A2 (Dyax Co.) as a template and the oligonucleotides VHAA1 (SEQ ID NO: 3) and GEN III-Rev (SEQ ID NO: 4) as primers. The amplified DNA product was subsequently digested with SfiI and NotI and cloned into an approximately 5.2 kb fragment derived from the digestion of pF11β (Cm^{R}) with SfiI-NotI (Veiga et al., Mol Microbiol 1999, 33 (6): 1232-43), under the control of the pLac promoter, rendering the plasmid pvamyβ (SEQ ID NO: 1). Said plasmid, pV_{amy}β, was digested with XbaI-HindIII and the approximately 1.9 kb fragment containing the V_{amy}β hybrid was cloned into plasmid pVLT35 (Sp^{R}) (Lorenzo et al., Gene 1993, 123 (1): 17-24), under the control of pTac promoter, giving the plasmid pVV_{amy}β.

### 1.2 Construction of the C-IgAP and 2V_{amy} hybrid protein (2V_{amy}β)

An approximately 0.4 kb DNA fragment encoding V_{amy} was amplified by PCR using the Linker-A48-V_{amy}A (SEQ ID NO: 5) and V_{amy}-NOt (SEQ ID NO: 6) primers of the phagemid A100R3A2 (see 1.1 above). The V_{amy} fragment was bound to the oligonucleotide Linker-A48 (SEQ ID NO: 7), which encodes the scFv A48III-1 flexible peptide (Proba et al., J. Mol. Biol. 1998, 275(2): 245-253), by a PCR reaction without primers, due to the homology between the first 24 bases of the Linker-A48-V_{amy}A and Linker-A48. This reaction was used for the amplification of the Linker-V_{amy} fusion product, using the oligonucleotides V_{amy}-Not (SEQ ID NO: 6) and Linker-A48-V_{amy}-eagl (SEQ ID NO: 8) as primers. The obtained PCR product (Linker-V_{amy}) was digested with NotI and cloned into plasmid pVV_{amy}β digested with the same enzyme. The new plasmid p2V_{amy}β encodes for a hybrid protein made up of two V_{HH} (V_{amy}β) bound by a flexible linker and fused to the C-IgAP. The amino acid sequence of said flexible linker is -TPSHNSHQVPSAGGPTANSG- (amino acid one-letter code).

### 1.3 Construction of the C-IgAP and 3V_{amy} hybrid protein (3V_{amy}β)

The product of the previous PCR reaction (Linker-V_{amy}) was digested with NotI and cloned into p2V_{amy}β digested with NotI. The new plasmid, called p3V_{amy}β, encoded a hybrid protein made up of three V_{HH} (V_{amy}β) bound by a flexible linker and fused to the C-IgAP. The amino acid sequence of said flexible linker is -TPSHNSHQVPSAGGPTANSG- (amino acid one-letter code).

### EXAMPLE 2

### Construction of pVLMB10β: V_{LMB10} domain fused to C-IgAP

A hybrid protein anchored to the external surface of the OM of *E. coli* comprising the V_{LMB10} domain fused to the C-IgAp domain, hereinafter referred to as V_{LMB10}β (Figure 3B), has been obtained. The signal peptide of PelB drives the secretion of said hybrid protein through the IM to the periplasmic space.

PCR was carried out with oligos VL1 (SEQ ID NO: 9) and VL2 (SEQ ID NO: 10) and using the pCES1-VLMB10 clone (van den Beucken et al., J. Mol. Biol. (2001) 310, 591-601) as template. The amplified 0.47 kb DNA fragment, containing the V_{L} domain, was digested with XbaI and NotI, and was cloned into plasmid pV_{amy}β, digested with the same enzymes, replacing the XbaI-NotI fragment which contains the V_{amy} domain in said plasmid. The constructed clone pVLMB10β (SEQ ID NO: 2), which expresses the hybrid protein V_{LMB10}β under the control of promoter pLac, was checked by means of DNA sequencing.

### EXAMPLE 3

### Expression of pVVamyβ in E. coli UT5600 cells

The expression of the V_{amy}β hybrid protein in *E*. *coli* UT5600 cells was analyzed in Western-blots using the anti-E-tag mAb (Figure 4). V_{amy}β was expressed in a stable manner as a 66 kDa protein, corresponding to the expected size for the fusion (Figure 4A, lane 1). However, the expression of FvHβ (Veiga et al., Mol. Microbiol. 1999, 33(6): 1232-1243), which gave rise not only to the complete 80 kDa protein, but rather to different bands of smaller sizes corresponding to the proteolytic degradation of the hybrid protein (Figure 4A, lane 2). Transformation of *E. coli* cells was carried out by conventional techniques (Ausubel et al., 1994. Current Protocols in Molecular Biology. John Wiley & Sons, New York; Sambrook et al., 1989. Molecular cloning. A laboratory manual. Cold Spring Harbor Laboratory Press, New York).

### EXAMPLE 4

### Exposure on the surface of E. coli and folding of the V_{amy}β passenger domains

The exposure on the surface of the V_{amy}β passenger domains was checked by means of the digestion of *E*. *coli* cells expressing V_{amy}β with trypsin. The samples were analyzed in Western-blot using the anti-E-tag mAb, or a rabbit anti-OmpA serum. In these experiments, the proteolysis of the V_{amy}β hybrid proteins in the presence of trypsin (Figure 4B) was checked, which indicates that these passengers were completely exposed on the *E*. *coli* surface. As previously described, the passenger of the FvHβ fusion was very weakly digested by the action of the trypsin (Figure 4B). The presence of the OmpA protein in all the lanes is shown as an internal loading control in the experiment. The absence of OmpA proteolysis (Figure 4B, lanes 3 and 7) shows the integrity of the OM. When the OM is artificially permeabilized with EDTA, there is an approximately 25 kDa band of OmpA proteolysis (T-OmpA) (Figure 4B, lanes 4 and 8).

In order to know the folding state of these chimeric passenger domains exposed towards the extracellular medium, the specific binding capacity of the Ig domains to their antigen was used. In order to assay binding activity, ELISA plates were covered with recognized antigens of the V_{amy}β passenger domain (α-amylase). BSA was used as an antigen control. *E*. *coli* cells which expressed the C-IgAP hybrids were incubated on ELISA plates and the binding to the Ig passenger was detected by means of the anti-E-tag mAb conjugated with POD. As is shown in Figure 5, the Ig passenger domain V_{amy}β exposed towards the extracellular medium exhibited specific binding activity to its antigen, which indicates that it was correctly folded. As a negative control of these experiments, *E. coli* cells expressing the HEβ hybrid protein were used, said hybrid protein consisting of a poly-His segment fused to the C-terminal β-domain of the *N. gonorrhoeae* IgA protease (Veiga et al., 2002, 21 (9) 2122-31).

### EXAMPLE 5

### Translocation of the 2V_{amy}β and 3V_{amy}β hybrid proteins

2V_{amy}β and 3V_{amy}β hybrid proteins were obtained as it is shown in Figure 7A. By means of proteolysis assays with trypsin, the translocation of the passenger domains (2V_{amy} and 3V_{amy}) was determined. It was found that the translocation of the 2V_{amy}β and 3V_{amy}β passenger domains was complete. In said figure, the DNA constructions encoding the hybrid proteins are shown, the signal peptide is removed during the passage of the protein across the periplasm and, finally, the hybrid proteins do not contain it.

### EXAMPLE 6

### Expression of pVLMB10β in E. coli UT5600 cells

*E. coli* UT5600 cells were transformed with pVLMB10β and were grown at 30°C in LB-agar plates (1.5% w/v) containing chloramphenicol (40 µg/ml) and glucose (2% w/v). Liquid LB cultures containing chloramphenicol (40 µg/ml) and glucose (2% w/v) were inoculated with these transformed clones, and they were grown at 30°C until the OD₆₀₀ was about 0.5. Then, the cells were harvested by centrifugation (4000xg, 5 min) and were resuspended in liquid LB medium containing chloramphenicol (40 µg/ml) and 0.1 mM IPTG for the induction of the pLac promoter. After 3 hours of incubation at 30°C, the induced cells were harvested by centrifugation and were analyzed by means of proteolysis with trypsin and Western-blot with anti-E-tag-POD.

Figure 6 shows a digestion with trypsin developed with anti-E-tag and OmpA (note that the trypsin lane is number 2). Exposure of over 90% of the V_{LMB10}β) fusion protein (65 kDa) was observed. A single, proteolytic degradation band of about 55 kDa, further appeared.

A peptidoglycan (PG) accessibility assay was also carried out, which included the control (pAKnot), V_{amy}β, 2V_{amy}β, 3V_{amy}β and V_{LMB10} constructs. In all of them, only a PG accessibility level appeared at about 5% maximum and identical to that observed in the control strain (see Figure 8) without expression of any hybrid protein.

### EXAMPLE 7

### Assay for specific adhesion to an antigen

In order to study the specific adhesion capacity of *E*. *coli* cells which expressed the hybrid proteins (comprising C-IgAP) in ELISA plates coated with recognized antigens of the passenger domain of the hybrid protein, *E. coli* UT5600 cells expressing the V_{amy}β hybrid protein were incubated in ELISA plates which had previously been coated with α-amylase antigen or control antigen (BSA) and the binding to the cells with a polyclonal anti-*E*. *coli* antibody generated in mice was revealed, and the binding of these antibodies with a mouse anti-IgGs antibody conjugated to peroxidase was detected (Figure 9). As a negative control, *E. coli* cells expressing the HEβ hybrid protein were used. The results obtained show the specific adhesion of bacteria having on the external surface of its OM a single-domain recombinant antibody (V_{amy}) in the form of a hybrid protein (V_{amy}β) to its antigen (α-amylase). Therefore, the hybrid protein V_{amy}β directs the adhesion of said bacteria depending on the antigen recognized by the Ig domain.

## Claims

1. A gene construction comprising:
a) a first nucleic acid sequence containing the nucleotide sequence encoding a signal peptide involved in the passage of proteins across the cell membrane;
b) a second nucleic acid sequence selected from the group consisting of:
i) the nucleic acid sequence encoding one single-domain recombinant antibody; and
ii) a nucleic acid sequence encoding two or more, equal, single-domain recombinant antibodies; and
c) a third nucleic sequence containing the nucleotide sequence encoding the C-terminal domain of an autotransporter capable of translocating passenger domain to which it is bound;
wherein the 3' end of said first nucleic acid sequence is linked to the 5' end of said second nucleic acid sequence and the 3' end of said second nucleic acid sequence is linked to the 5' end of said third nucleic acid sequence.

2. Construction according to claim 1, wherein said signal peptide is selected from the signal peptide of PelB, the signal peptide of OmpA, the signal peptide of the M13 bacteriophage protein 3 and the signal peptide of the maltose binding protein (MBP).

3. Construction according to claim 1, wherein said single-domain recombinant antibody is selected from a natural or modified heavy chain variable domain (V_{H}) of an antibody, a natural or modified light chain variable domain (V_{L}) of an antibody, a natural or modified recombinants camelide antibody (V_{HH}), a humanized recombinant camelide antibody, a single-domain recombinant antibody of a non-camelide animal capable of interacting in the form of a single-domain with its antigen, and an IgNAR single-domain antibody of cartilaginous fish.

4. Construction according to claim 1 or 3, wherein said second nucleic acid sequence comprises the nucleotide sequences encoding two or more, equal, single-domain recombinant antibodies.

5. Construction according to claim 4, wherein said second nucleic acid sequence comprises the nucleotide sequences encoding three, equal, single-domain recombinant antibodies.

6. Construction according to claim 4 or 5, wherein said nucleotide sequences encoding the single-domain recombinant antibodies are separated from each other by nucleic acid sequences encoding a spacer placed between two of said nucleotide sequences encoding the single-domain recombinant antibodies.

7. Construction according to claim 6, wherein said spacer is a peptide sequence having structural flexibility.

8. Construction according to claim 7, wherein said peptide sequence having structural flexibility comprises an amino acid residues multimer or the hinge region of an antibody.

9. Construction according to claim 1, wherein said third nucleic acid sequence comprises the nucleotide sequence encoding the C-terminal domain of an AT of a gram-negative bacteria.

10. Construction according to claim 9, wherein said third nucleic acid sequence comprises the nucleotide sequence encoding the C-terminal β-domain of *Neisseria gonorrhoeae* IgA protease.

11. Construction according to claim 1, which further comprises a fourth nucleic acid sequence encoding a spacer placed between said second and third nucleic acid sequences, wherein the 5' end of said fourth nucleic acid sequence is linked to the 3' end of said second nucleic acid sequence and the 3' end of said fourth nucleic acid sequence is linked to the 5' end of said third nucleic acid sequence.

12. Construction according to claim 11, wherein said spacer is a peptide sequence having structural flexibility.

13. Construction according to claim 12, wherein said peptide sequence having structural flexibility comprises an amino acid residues multimer or the hinge region of an antibody.

14. Construction according to claim 1, which further comprises a fifth nucleic acid sequence encoding a peptide sequence for detection purposes.

15. Construction according to claim 14, wherein said peptide sequence for detection purposes is a peptide sequence susceptible of being recognized by an antibody.

16. Construction according to claim 15, wherein said peptide sequence susceptible of being recognized by an antibody comprises a poly-hystidine sequence, the E-epitope sequence, the HA epitope sequence, the FLAG epitope sequence or the c-myc epitope sequence.

17. Construction according to claim 14, wherein said fifth nucleic acid sequence is placed between said second and third nucleic acid sequences, wherein the 5' end of said fifth nucleic acid sequence is linked to the 3' end of said second nucleic acid sequence and the 3' end of said fifth nucleic acid sequence is linked to the 5' end of said third nucleic acid sequence.

18. An expression vector comprising a gene construction according to anyone of claims 1 to 17 operatively linked to a transcription control sequence.

19. Vector according to claim 18, wherein said transcription control sequence comprises a promoter, a sequence encoding transcriptional regulators, a ribosome binding sequence and/or a transcription terminator sequence.

20. Vector according to claim 19, wherein said transcription control sequence is functional in bacteria and comprises a transcription promoter functional in bacteria, inducible or constitutive.

21. Vector according to claim 20, wherein said promoter is selected from promoter pTetA, promoter pBAD, the promoter of *Escherichia coli lac* operon (pLac), promoter pTac, promoter Pm and the promoter of *E*. *coli* β-lactamase.

22. Vector according to claim 18, further comprising a marker.

23. A bacterial cell comprising a gene construction according to anyone of claims 1 to 17 or an expression vector according to anyone of claims 18 to 22.

24. Bacterial cell according to claim 23, **characterized in that** is a gram-negative bacteria.

25. Bacterial cell according to claim 24, selected from a *Escherichia spp.* strain, a *Salmonella spp.* strain and a *Pseudomonas spp.* strain.

26. A hybrid protein obtainable by the expression of the nucleic acid sequence contained in a gene construction according to anyone of claims 1 to 17 or in an expression vector according to anyone of claims 18 to 22.

27. Hybrid protein according to claim 26, comprising a domain (A) which comprises the amino acid sequence of, at least, a single-domain recombinant antibody, and a domain (B) comprising the amino acid sequence of the C-terminal domain of an AT.

28. Hybrid protein according to claim 27, wherein said domain (B) comprises the amino acid sequence of the C-terminal β-domain of *Neisseria gonorrhoeae* IgA protease.

29. Hybrid protein according to claim 27 or 28, further comprising a spacer between said domains (A) and (B) and/or a peptide sequence for detection purposes of said hybrid protein.

30. A method of anchoring and expressing a single-domain recombinant antibody on the surface of the outer membrane (OM) of a bacterial cell which comprises culturing a bacterial cell according to anyone of claims 23 to 25, under conditions which allow for the production of said single-domain recombinant antibody, its anchoring and expression on the surface of the OM of said bacterial cell in the form of a hybrid protein.

31. A method for the specific adhesion of a bacterial cell to an antigen which comprises the steps of:
a) transforming a bacterial cell with a gene construction according to anyone of claims 1 to 17 or with an expression vector according to anyone of claims 18 to 22, said gene construction or expression vector comprising the nucleotide sequence encoding a single-domain recombinant antibody capable of recognizing said antigen;
b) culturing said transformed bacterial cell under conditions which allow for the production of said single-domain recombinant antibody, its anchoring and expression on the surface of the outer membrane (OM) of said bacterial cell; and
c) contacting the transformed bacterial cell and cultured in step b) with said antigen.

32. A method for producing a single-domain recombinant antibody anchored on the surface of the outer membrane (OM) of a bacterial cell which comprises culturing a bacterial cell according to anyone of claims 23 to 25, under conditions which allow for the production of said single-domain recombinant antibody, its anchoring and expression on the surface of the OM of said bacterial cell in the form of a hybrid protein.

33. Method according to anyone of claims 30 to 32 wherein said bacterial cell is a gram-negative bacterial cell.

## Patentansprüche

1. Genkonstrukt umfassend:
a) eine erste Nucleinsäuresequenz enthaltend die Nucleotidsequenz, die ein Signalpeptid codiert, welches an dem Durchtritt von Proteinen durch die Zellmembran beteiligt ist;
b) eine zweite Nucleinsäuresequenz ausgewählt aus der Gruppe bestehend aus:
i) der Nucleinsäuresequenz, die einen rekombinanten Einzeldomänen-Antikörper codiert; und
ii) einer Nucleinsäuresequenz, die zwei oder mehr gleiche rekombinante Einzeldomänen-Antikörper codiert; und
c) eine dritte Nucleinsäureseqenz enthaltend die Nucleotidsequenz, die die C-terminale Domäne eines Autotransporters codiert, der in der Lage ist, die Passenger-Domäne, an die sie gebunden ist, zu translozieren;
wobei das 3'-Ende der ersten Nucleinsäuresequenz mit dem 5'-Ende der zweiten Nucleinsäuresequenz verbunden ist und das 3'-Ende der zweiten Nucleinsäuresequenz mit dem 5'-Ende der dritten Nucleinsäuresequenz verbunden ist.

2. Konstrukt gemäß Anspruch 1, wobei das Signalpeptid ausgewählt ist aus dem Signalpeptid von PelB, dem Signalpeptid von OmpA, dem Signalpeptid von dem M13-Bakteriophagenprotein 3 und dem Signalpeptid von dem Maltose-bindenden Protein (MBP).

3. Konstrukt gemäß Anspruch 1, wobei der rekombinante Einzeldomänen-Antikörper ausgewählt ist aus einer natürlichen oder modifizierten variablen Domäne einer schweren Kette eines Antikörpers (V_{H}), einer natürlichen oder modifizierten variablen Domäne einer leichten Kette eines Antikörpers (V_{L}), einem natürlichen oder modifizierten rekombinanten cameliden Antikörper (V_{HH}), einem humanisierten rekombinanten cameliden Antikörper, einem rekombinanten Einzeldomänen-Antikörper eines nicht-cameliden Tieres, der in der Lage ist, in der Form einer Einzeldomäne mit seinem Antigen zu interagieren, und einem IgNAR-Einzeldomänen-Antikörper von Knorpelfisch.

4. Konstrukt gemäß Anspruch 1 oder 3, wobei die zweite Nucleinsäuresequenz die Nucleotidsequenzen umfasst, die zwei oder mehr gleiche rekombinante Einzeldomänen-Antikörper codieren.

5. Konstrukt gemäß Anspruch 4, wobei die zweite Nucleinsäuresequenz die Nucleotidsequenzen umfasst, die drei gleiche rekombinante Einzeldomänen-Antikörper codieren.

6. Konstrukt gemäß Anspruch 4 oder 5, wobei die Nucleotidsequenzen, die die rekombinanten Einzeldomänen-Antikörper codieren, voneinander durch Nucleinsäuresequenzen getrennt sind, die einen Spacer codieren, der zwischen zwei der Nucleotidsequenzen, die die rekombinanten Einzeldomänen-Antikörper codieren, platziert ist.

7. Konstrukt gemäß Anspruch 6, wobei der Spacer eine Peptidsequenz ist, die strukturelle Flexibilität hat.

8. Konstrukt gemäß Anspruch 7, wobei die Peptidsequenz, die strukturelle Flexibilität hat, ein Aminosäurereste-Multimer oder die Gelenkregion eines Antikörpers umfasst.

9. Konstrukt gemäß Anspruch 1, wobei die dritte Nucleinsäuresequenz die Nucleotidsequenz umfasst, die die C-terminale Domäne von einem AT eines Gram-negativen Bakteriums codiert.

10. Konstrukt gemäß Anspruch 9, wobei die dritte Nucleinsäuresequenz die Nucleotidsequenz umfasst, die die C-terminale β-Domäne der Neisseria gonorrhoeae-IgA-Protease codiert.

11. Konstrukt gemäß Anspruch 1, welches weiter eine vierte Nucleinsäuresequenz umfasst, die einen Spacer codiert, der zwischen der zweiten und dritten Nucleinsäuresequenz platziert ist, wobei das 5 '-Ende der vierten Nucleinsäuresequenz mit dem 3'-Ende der zweiten Nucleinsäuresequenz verbunden ist und das 3'-Ende der vierten Nucleinsäuresequenz mit dem 5'-Ende der dritten Nucleinsäuresequenz verbunden ist.

12. Konstrukt gemäß Anspruch 11, wobei der Spacer eine Peptidsequenz ist, die strukturelle Flexibilität hat.

13. Konstrukt gemäß Anspruch 12, wobei die Peptidsequenz, die strukturelle Flexibilität hat, ein Aminosäurereste-Multimer oder die Gelenkregion eines Antikörpers umfasst.

14. Konstrukt gemäß Anspruch 1, welche weiter eine fünfte Nucleinsäuresequenz umfasst, die eine Peptidsequenz zu Nachweiszwecken codiert.

15. Konstrukt gemäß Anspruch 14, wobei die Peptidsequenz zu Nachweiszwecken eine Peptidsequenz ist, die fähig ist, von einem Antikörper erkannt zu werden.

16. Konstrukt gemäß Anspruch 15, wobei die Peptidsequenz, die fähig ist, von einem Antikörper erkannt zu werden, eine Poly-Histidin-Sequenz, die E-Epitop-Sequenz, die HA-Epitop-Sequenz, die FLAG-Epitop-Sequenz oder die c-myc-Epitop-Sequenz umfasst.

17. Konstrukt gemäß Anspruch 14, wobei die fünfte Nucleinsäuresequenz zwischen der zweiten und dritten Nucleinsäuresequenz platziert ist, wobei das 5'-Ende der fünften Nucleinsäuresequenz mit dem 3'-Ende der zweiten Nucleinsäuresequenz verbunden ist und das 3'-Ende der fünften Nucleinsäuresequenz mit dem 5'-Ende der dritten Nucleinsäuresequenz verbunden ist.

18. Expressionsvektor der ein Genkonstrukt gemäß einem der Ansprüche 1 bis 17 umfasst, das funktionell mit einer Transkriptionskontrollsequenz verknüpft ist.

19. Vektor gemäß Anspruch 18, wobei die Transkriptionskontrollsequenz einen Promotor, eine Sequenz, die Transkriptionsregulatoren codiert, eine Ribosomenbindesequenz und/oder eine Transkriptionsterminatorsequenz umfasst.

20. Vektor gemäß Anspruch 19, wobei die Transkriptionskontrollsequenz in Bakterien funktioniert und einen Transkriptionspromotor umfasst, der in Bakterien funktioniert, induzierbar oder konstitutiv.

21. Vektor gemäß Anspruch 20, wobei der Promotor ausgewählt ist aus Promotor pTetA, Promotor pBAD, dem Promotor des *Escherichia coli-lac-*Operons (pLac), Promotor pTac, Promotor Pm und dem Promotor von *E*. *coli*-β-Lactamase.

22. Vektor gemäß Anspruch 18, der des Weiteren einen Marker umfasst.

23. Bakterienzelle, die ein Genkonstrukt gemäß einem der Ansprüche 1 bis 17 oder einen Expressionsvektor gemäß einem der Ansprüche 18 bis 22 umfasst.

24. Bakterienzelle gemäß Anspruch 23, **dadurch gekennzeichnet, dass** es ein Gramnegatives Bakterium ist.

25. Bakterienzelle gemäß Anspruch 24, ausgewählt aus einem *Escherichia spp.-*Stamm, einem *Salmonella spp.-Stamm* und einem *Pseudomonas spp.*-Stamm.

26. Hybridprotein erhältlich durch die Expression der Nucleinsäuresequenz, die in einem Genkonstrukt gemäß einem der Ansprüche 1 bis 17 oder in einem Expressionsvektor gemäß einem der Ansprüche 18 bis 22 enthalten ist.

27. Hybridprotein gemäß Anspruch 26, umfassend eine Domäne (A), welche die Aminosäuresequenz von mindestens einem rekombinanten Einzeldomänen-Antikörper umfasst, und eine Domäne (B), welche die Aminosäuresequenz der C-terminalen Domäne von einem AT umfasst.

28. Hybridprotein gemäß Anspruch 27, wobei die Domäne (B) die Aminosäuresequenz der C-terminalen β-Domäne der *Neisseria gonorrhoeae-*IgA-Protease umfasst.

29. Hybridprotein gemäß Anspruch 27 oder 28, das des Weiteren einen Spacer zwischen den Domänen (A) und (B) und/oder eine Peptidsequenz zu Nachweiszwecken des Hybridproteins umfasst.

30. Verfahren zur Verankerung und Expression eines rekombinanten Einzeldomänen-Antikörpers an der Oberfläche der äußeren Membran (OM) einer Bakterienzelle, umfassend das Züchten einer Bakterienzelle gemäß einem der Ansprüche 23 bis 25 unter Bedingungen, die die Produktion des rekombinanten Einzeldomänen-Antikörpers, seine Verankerung und die Expression an der Oberfläche der OM der Bakterienzelle in der Form eines Hybridproteins erlauben.

31. Verfahren für die spezifische Adhäsion einer Bakterienzelle an ein Antigen, welches die Schritte umfasst:
a) Transformieren einer Bakterienzelle mit einem Genkonstrukt gemäß einem der Ansprüche 1 bis 17 oder mit einem Expressionsvektor gemäß einem der Ansprüche 18 bis 22, wobei das Genkonstrukt oder der Expressionsvektor die Nucleotidsequenz umfasst, die einen rekombinanten Einzeldomänen-Antikörper codiert, der in der Lage ist, das Antigen zu erkennen;
b) Züchten der transformierten Bakterienzelle unter Bedingungen, die die Produktion des rekombinanten Einzeldomänen-Antikörpers, seine Verankerung und Expression an der Oberfläche der äußeren Membran (OM) der Bakterienzelle erlauben; und
c) Inkontaktbringen der transformierten und in Schritt b) kultivierten Bakterienzelle mit dem Antigen.

32. Verfahren zur Herstellung eines rekombinanten Einzeldomänen-Antikörpers, der an der Oberfläche der äußeren Membran (OM) einer Bakterienzelle verankert ist, umfassend das Züchten einer Bakterienzelle gemäß einem der Ansprüche 23 bis 25 unter Bedingungen, die die Produktion des rekombinanten Einzeldomänen-Antikörpers, seine Verankerung und die Expression an der Oberfläche der OM der Bakterienzelle in der Form eines Hybridproteins erlauben.

33. Verfahren gemäß einem der Ansprüche 30 bis 32, wobei die Bakterienzelle eine Gram-negative Bakterienzelle ist.

## Revendications

1. Construction de gène, comprenant :
a) une première séquence d'acides nucléiques codant pour un peptide signal intervenant dans le passage de protéines à travers la membrane cellulaire ;
b) une deuxième séquence d'acides nucléiques, choisie parmi le groupe consistant en :
i) la séquence d'acides nucléiques codant pour un anticorps recombinant à un seul domaine ; et
ii) une séquence d'acides nucléiques codant pour deux ou plus anticorps recombinants égaux, à un seul domaine; et
c) une troisième séquence nucléique contenant la séquence de nucléotides codant pour le domaine C-terminal d'un autotransporteur pouvant assurer la translocation d'un domaine passager auquel il est lié ;
dans laquelle l'extrémité 3' de ladite première séquence d'acides nucléiques est liée à l'extrémité 5' de ladite deuxième séquence d'acides nucléiques, et l'extrémité 3' de ladite deuxième séquence d'acides nucléiques est liée à l'extrémité 5' de ladite troisième séquence d'acides nucléiques.

2. Construction selon la revendication 1, dans laquelle ledit peptide signal est choisi parmi le peptide signal de PelB, le peptide signal de OmpA, le peptide signal de la protéine 3 du bactériophage M13 et le peptide signal de la protéine liant le maltose (MBP).

3. Construction selon la revendication 1, dans laquelle ledit anticorps recombinant à un seul domaine est choisi parmi un domaine variable de chaîne lourde (V_{H}) naturel ou modifié d'un anticorps, un domaine variable de chaîne légère (V_{L}) naturel ou modifié d'un anticorps, un anticorps de camélidé recombinant (V_{HH}) naturel ou modifié, un anticorps de camélidé recombinant humanisé, un anticorps recombinant à un seul domaine d'un animal non camélidé, pouvant interagir sous la forme d'un seul domaine avec son antigène, et un anticorps IgNAR à un seul domaine de poisson cartilagineux.

4. Construction selon la revendication 1 ou 3, dans laquelle ladite deuxième séquence d'acides nucléiques comprend les séquences de nucléotides codant pour deux ou plus anticorps recombinants égaux, à un seul domaine.

5. Construction selon la revendication 4, dans laquelle ladite deuxième séquence d'acides nucléiques comprend les séquences de nucléotides codant pour trois anticorps recombinants égaux, à un seul domaine.

6. Construction selon la revendication 4 ou 5, dans laquelle lesdites séquences de nucléotides codant pour les anticorps recombinants à un seul domaine, sont séparées les unes des autres par des séquences d'acides nucléiques codant pour un espaceur situé entre deux desdites séquences de nucléotides codant pour les anticorps recombinants à un seul domaine.

7. Construction selon la revendication 6, dans laquelle ledit espaceur est une séquence peptidique ayant une souplesse structurale.

8. Construction selon la revendication 7, dans laquelle ladite séquence peptidique ayant une souplesse structurale, comprend un multimère de résidus d'aminoacide, ou la région charnière d'un anticorps.

9. Construction selon la revendication 1, dans laquelle ladite troisième séquence d'acides nucléiques, comprend la séquence de nucléotides codant pour le domaine C-terminal d'un autotransporteur (AT) d'une bactérie Gram négatif.

10. Construction selon la revendication 9, dans laquelle ladite troisième séquence d'acides nucléiques comprend la séquence de nucléotides codant pour le domaine β C-terminal de la protéase IgA de *Neisseria gonorrhoeae.*

11. Construction selon la revendication 1, qui comprend, en outre, une quatrième séquence d'acides nucléiques codant pour un espaceur situé entre lesdites deuxième et troisième séquences d'acides nucléiques, dans laquelle l'extrémité 5' de ladite quatrième séquence d'acides nucléiques est liée à l'extrémité 3' de ladite deuxième séquence d'acides nucléiques, et l'extrémité 3' de ladite quatrième séquence d'acides nucléiques est liée à l'extrémité 5' de ladite troisième séquence d'acides nucléiques.

12. Construction selon la revendication 11, dans laquelle ledit espaceur est une séquence peptidique ayant une souplesse structurale.

13. Construction selon la revendication 12, dans laquelle ladite séquence peptidique ayant une souplesse structurale, comprend un multimère de résidus d'aminoacide, ou la région charnière d'un anticorps.

14. Construction selon la revendication 1, qui comprend, en outre, une cinquième séquence d'acides nucléiques codant pour une séquence peptidique destinée à des applications de détection.

15. Construction selon la revendication 14, dans laquelle ladite séquence peptidique, destinée à des applications de détection, est une séquence peptidique susceptible d'être reconnue par un anticorps.

16. Construction selon la revendication 15, dans laquelle ladite séquence peptidique susceptible d'être reconnue par un anticorps, comprend une séquence poly-histidine, la séquence d'épitope E, la séquence d'épitope HA, la séquence d'épitope FLAG ou la séquence d'épitope c-myc.

17. Construction selon la revendication 14, dans laquelle ladite cinquième séquence d'acides nucléiques est située entre lesdites deuxième et troisième séquences d'acides nucléiques, dans laquelle l'extrémité 5' de ladite cinquième séquence d'acides nucléiques est liée à l'extrémité 3' de ladite deuxième séquence d'acides nucléiques, et dans laquelle l'extrémité 3' de ladite cinquième séquence d'acides nucléiques est liée à l'extrémité 5' de ladite troisième séquence d'acides nucléiques.

18. Vecteur d'expression, comprenant une construction de gène selon l'une quelconque des revendications 1 à 17, fonctionnellement liée à une séquence de contrôle de transcription.

19. Vecteur selon la revendication 18, dans lequel ladite séquence de contrôle de transcription comprend un promoteur, une séquence codant pour des régulateurs de transcription, une séquence de liaison à un ribosome et/ou une séquence de terminaison de transcription.

20. Vecteur selon la revendication 19, dans lequel ladite séquence de contrôle de transcription est fonctionnelle dans une bactérie, et comprend un promoteur de transcription fonctionnelle dans une bactérie, inductible ou constitutif.

21. Vecteur selon la revendication 20, dans lequel ledit promoteur est choisi parmi le promoteur PTetA, le promoteur pBAD, le promoteur de l'opéron lac d'*Escherichia coli* (pLac), le promoteur pTac, le promoteur Pm et le promoteur de la β-lactamase d'*E.coli.*

22. Vecteur selon la revendication 18, comprenant, en outre, un marqueur.

23. Cellule bactérienne comprenant une construction de gène selon l'une quelconque des revendications 1 à 17, ou un vecteur d'expression selon l'une quelconque des revendications de 18 à 22.

24. Cellule bactérienne selon la revendication 23, **caractérisée en ce qu'**elle est une bactérie Gram négatif.

25. Cellule bactérienne selon la revendication 24, choisie parmi une souche du genre *Escherichia,* une souche du genre *Salmonella* et une souche du genre *Pseudomonas.*

26. Protéine hybride pouvant être obtenue par expression de la séquence d'acides nucléiques contenue dans une construction de gène selon l'une quelconque des revendications 1 à 17, ou dans un vecteur d'expression selon l'une quelconque des revendications 18 à 22.

27. Protéine hybride selon la revendication 26, comprenant un domaine (A) qui comprend la séquence d'aminoacides d'au moins un anticorps recombinant à un seul domaine, et un domaine (B) comprenant la séquence d'aminoacides du domaine C-terminal d'un AT.

28. Protéine hybride selon la revendication 27, dans laquelle ledit domaine (B) comprend la séquence d'aminoacides du domaine β C-terminal de la protéase IgA de *Neisseria gonorrhoeae.*

29. Protéine hybride selon la revendication 27 ou 28, comprenant, en outre, un espaceur entre lesdits domaines (A) et (B) et/ou une séquence peptidique destinée à des applications de détection de ladite protéine hybride.

30. Procédé d'ancrage et d'expression d'un anticorps recombinant à un seul domaine, sur la surface de la membrane extérieure (OM) d'une cellule bactérienne, qui comprend la culture d'une cellule bactérienne selon l'une quelconque des revendications 23 à 25, dans des conditions qui permettent la production du dit anticorps recombinant à un seul domaine, son ancrage et son expression à la surface de l'OM de ladite cellule bactérienne, sous la forme d'une protéine hybride.

31. Procédé pour l'adhésion spécifique d'une cellule bactérienne à un antigène, qui comprend les étapes consistant à :
a) transformer une cellule bactérienne avec une construction de gène selon l'une quelconque des revendications 1 à 17, ou avec un vecteur d'expression selon l'une quelconque des revendications 18 à 22, ladite construction de gène ou ledit vecteur d'expression, comprenant la séquence de nucléotides codant pour un anticorps recombinant à un seul domaine, pouvant reconnaître ledit antigène ;
b) cultiver ladite cellule bactérienne transformée, dans des conditions qui permettent la production du dit anticorps recombinant à un seul domaine, son ancrage et son expression à la surface de la membrane extérieure (OM) de ladite cellule bactérienne ; et
c) mettre en contact la cellule bactérienne transformée et cultivée dans l'étape b), avec ledit antigène.

32. Procédé pour la production d'un anticorps recombinant à un seul domaine, ancré à la surface de la membrane extérieure (OM) d'une cellule bactérienne, qui comprend la culture d'une cellule bactérienne selon l'une quelconque des revendications 23 à 25, dans des conditions qui permettent la production du dit anticorps recombinant à un seul domaine, son ancrage et son expression à la surface de l'OM de ladite cellule bactérienne, sous la forme d'une protéine hybride.

33. Procédé selon l'une quelconque des revendications 30 à 32, dans lequel ladite cellule bactérienne est une cellule bactérienne Gram négatif.


### SEQUENCE LISTING

<110> Consejo Superior de Investigaciones Científicas
<120> GENERATION OF SPECIFIC ADHESION IN GRAM-NEGATIVE BACTERIA BY MEANS OF FIXING IMMUNOGLOBULIN SINGLE DOMAINS ON THEIR SURFACE WITH AUTOTRANSPORTERS
<130> P1375PC
<150> ES P200400073 <151> 2004-01-14 (January 14, 2004)
<160> 10
<170> PatentIn version 3.1
<210> 1
   <211> 5587
   <212> DNA
   <213> Artificial
<223> DNA sequence of plasmid pVamyβ
<400> 1
   acccgacacc atcgaatggc gcaaaacctt tcgcggtatg gcatgatagc gcccggaaga     60
   gagtcaattc agggtggtga atgtgaaacc agtaacgtta tacgatgtcg cagagtatgc    120
   cggtgtctct tatcagaccg tttcccgcgt ggtgaaccag gccagccacg tttctgcgaa    180
   aacgcgggaa aaagtggaag cggcgatggc ggagctgaat tacattccca accgcgtggc    240
   acaacaactg gcgggcaaac agtcgttgct gattggcgtt gccacctcca gtctggccct    300
   gcacgcgccg tcgcaaattg tcgcggcgat taaatctcgc gccgatcaac tgggtgccag    360
   cgtggtggtg tcgatggtag aacgaagcgg cgtcgaagcc tgtaaagcgg cggtgcacaa    420
   tcttctcgcg caacgcgtca gtgggctgat cattaactat ccgctggatg accaggatgc    480
   cattgctgtg gaagctgcct gcactaatgt tccggcgtta tttcttgatg tctctgacca    540
   gacacccatc aacagtatta ttttctccca tgaagacggt acgcgactgg gcgtggagca    600
   tctggtcgca ttgggtcacc agcaaatcgc gctgttagcg ggcccattaa gttctgtctc    660
   ggcgcgtctg cgtctggctg gctggcataa atatctcact cgcaatcaaa ttcagccgat    720
   agcggaacgg gaaggcgact ggagtgccat gtccggtttt caacaaacca tgcaaatgct    780
   gaatgagggc atcgttccca ctgcgatgct ggttgccaac gatcagatgg cgctgggcgc    840
   aatgcgcgcc attaccgagt ccgggctgcg cgttggtgcg gacatctcgg tagtgggata    900
   cgacgatacc gaagacagct catgttatat cccgccgtta accaccatca aacaggattt    960
   tcgcctgctg gggcaaacca gcgtggaccg cttgctgcaa ctctctcagg gccaggcggt   1020
   gaagggcaat cagctgttgc ccgtctcact ggtgaaaaga aaaaccaccc tggcgcccaa   1080
   tacgcaaacc gcctctcccc gcgcgttggc cgattcatta atgcagctgg cacgacaggt   1140
   ttcccgactg gaaagcgggc agtgagcggt acccgataaa agcggcttcc tgacaggagg   1200
   ccgttttgtt ttgcagccca cctcaacgca attaatgtga gttagctcac tcattaggca   1260
   ccccaggctt tacactttat gcttccggct cgtatgttgt gtggaattgt gagcggataa   1320
   caatttcaca caggaaacag ctatgaccat gattacgaat ttctagataa cgagggcaaa   1380
   tcatgaaata cctattgcct acggcagccg ctggattgtt attactcgcg gcccagccgg   1440
   ccatggctca ggtgcagctg gtggagtctt ggggaggctc ggtgcaggct ggggggtctc    1500
   tgagactctc ctgcacagcc cctggattca cctccaatag ctgccgcatg gactggtacc   1560
   gccaggctgc agggaagcag cgcgagtggg tctcatctat tagtactgat ggtcgcacaa   1620
   gctatgcaga ctccgtgaag ggccgattca ccatctccaa agacaaagcc aaggacacgg   1680
   tgtatctgca aatgaacagc ctgaaacctg aggacacggc catctattac tgtgccgtga   1740
   ggacgaatgg gtatcgtccg caatctcacg aatttcgcta ctggggcccg gggacccagg   1800
   tcaccgtctc ctcagcggcc gcggcgtcgg gggccgaatt cgtcgacggt gcgccggtgc   1860
   cgtatccgga tccgctggaa ccgatcgaca attcagccgc aattagtatg gcaaatccac   1920
   gtccaccaac accgcgggtc gctgcggccg tattttcatt ggatgattat gatgcaaaag   1980
   acaatagtga atcatcaata ggtaatttag ctcgtgtaat acctagaatg ggaagggagt   2040
   taattaatga ttatgaagaa atccccttgg aggagttgga agatgaagcg gaagaagaac   2100
   gtcgccaagc aacgcaattc cactccaaaa gtcgtaaccg tagagctata tcatcggaac 2160
   catcatctga tgaagatgca tctgaatcgg tttccacatc agacaaacac cctcaagata 2220
   atacggaact tcatgaaaaa gttgagacgg cgggtttaca accaagagcc gcgcagccgc 2280
   gaacccaagc cgccgcgcaa gccgatgcag tcagcaccaa tactaactcg gctttatctg 2340
   acgcaatggc aagcacgcaa tctatcttgt tggatacagg tgcttactta acacggcaca 2400
   ttgcacaaaa atcacgcgct gatgccgaaa aaaacagtgt ttggatgtca aacaccggtt 2460
   atggccgtga ttatgcttcc gcacaatatc gccggtttag ttcgaaacgc acgcaaacac 2520
   aaatcggcat tgaccgcagc ttgtccgaaa atatgcagat aggcggagta ttgacttact 2580
   ctgacagtca gcatactttt gatcaggcgg gcggcaaaaa tacttttgtg caagccaacc 2640
   tttatggtaa gtattattta aatgatgctt ggtatgtggc cggcgatatt ggtgcgggca 2700
   gcttgagaag ccggttacaa acgcagcaaa aagcaaactt taaccgaaca agcatccaaa 2760
   ccggccttac tttgggcaat acgctgaaaa tcaatcaatt cgagattgtc cctagtgcgg 2820
   gtatccgtta cagccgcctg tcatctgcag attacaagtt gggtgacgac agtgttaaag 2880
   taagttctat ggcagtgaaa acactaacgg ccggactgga ttttgcttat cggtttaaag 2940
   tcggcaacct taccgtaaaa cccttgttat ctgcagctta ctttgccaat tatggcaaag 3000
   gcggcgtgaa tgtgggcggt aaatccttcg cctataaagc agataatcaa cagcaatatt 3060
   cagcaggcgt cgcgttactg taccgtaatg ttacattaaa cgtaaatggc agtattacaa 3120
   aaggaaaaca attggaaaaa caaaaatccg gacaaattaa aatacagatt cgtttctaaa 3180
   ataccaaatt catagcaaaa taaaatgccg tctgaactca agcttgacct gtgaagtgaa 3240
   aaatggcgca cattgtgcga catttttttt gtctgccgtt taccgctact gcgtcacgga 3300
   tccccacgcg ccctgtagcg gcgcattaag cgcggcgggt gtggtggtta cgcgcagcgt 3360
   gaccgctaca cttgccagcg ccctagcgcc cgctcctttc gctttcttcc cttcctttct 3420
   cgccacgttc gccggctttc cccgtcaagc tctaaatcgg ggcatccctt tagggttccg 3480
   atttagtgct ttacggcacc tcgaccccaa aaaacttgat tagggtgatg gttcacgtag 3540
   tgggccatcg ccctgataga cggtttttcg ccctttgacg ttggagtcca cgttctttaa 3600
   tagtggactc ttgttccaaa ctggaacaac actcaaccct atctcggtct attcttttga 3660
   tttataaggg attttgccga tttcggccta ttggttaaaa aatgagctga tttaacaaaa 3720
   atttaacgcg aattttaaca aaatattaac gtttacaatt tcaggtggca cttttcgggg 3780
   aaatgtgcgc ggaaccccta tttgtttatt tttctaaata cattcaaata tgtatccgct 3840
   catgtcgaga cgttgggtga ggttccaact ttcaccataa tgaaataaga tcactaccgg 3900
   gcgtattttt tgagttatcg agattttcag gagctaagga agctaaaatg gagaaaaaaa 3960
   tcactggata taccaccgtt gatatatccc aatggcatcg taaagaacat tttgaggcat 4020
   ttcagtcagt tgctcaatgt acctataacc agaccgttca gctggatatt acggcctttt 4080
   taaagaccgt aaagaaaaat aagcacaagt tttatccggc ctttattcac attcttgccc 4140
   gcctgatgaa tgctcatccg gagttccgta tggcaatgaa agacggtgag ctggtgatat 4200
   gggatagtgt tcacccttgt tacaccgttt tccatgagca aactgaaacg ttttcatcgc 4260
   tctggagtga ataccacgac gatttccggc agtttctaca catatattcg caagatgtgg 4320
   cgtgttacgg tgaaaacctg gcctatttcc ctaaagggtt tattgagaat atgtttttcg 4380
   tctcagccaa tccctgggtg agtttcacca gttttgattt aaacgtggcc aatatggaca 4440
   acttcttcgc ccccgttttc accatgggca aatattatac gcaaggcgac aaggtgctga 4500
   tgccgctggc gattcaggtt catcatgccg tctgtgatgg cttccatgtc ggcagaatgc 4560
   ttaatgaatt acaacagtac tgcgatgagt ggcagggcgg ggcgtaattt ttttaaggca 4620
   gttattggtg cccttaaacg cctggtgcta cgcctgaata agtgataata agcggatgaa 4680
   tggcagaaat tcgaaagcaa attcgacccg gtcgtcggtt cagggcaggg tcgttaaata 4740
   gccgcttatg tctattgctg gtttaccggt ttattgacta ccggaagcag tgtgaccgtg 4800
   tgcttctcaa atgcctgagg ccagtttgct caggctctcc ccgtggaggt aataattgct 4860
   cgacatgacc aaaatccctt aacgtgagtt ttcgttccac tgagcgtcag accccgtaga 4920
   aaagatcaaa ggatcttctt gagatccttt ttttctgcgc gtaatctgct gcttgcaaac 4980
   aaaaaaacca ccgctaccag cggtggtttg tttgccggat caagagctac caactctttt 5040
   tccgaaggta actggcttca gcagagcgca gataccaaat actgtccttc tagtgtagcc 5100
   gtagttaggc caccacttca agaactctgt agcaccgcct acatacctcg ctctgctaat 5160
   cctgttacca gtggctgctg ccagtggcga taagtcgtgt cttaccgggt tggactcaag 5220
   acgatagtta ccggataagg cgcagcggtc gggctgaacg gggggttcgt gcacacagcc 5280
   cagcttggag cgaacgacct acaccgaact gagataccta cagcgtgagc tatgagaaag 5340
   cgccacgctt cccgaaggga gaaaggcgga caggtatccg gtaagcggca gggtcggaac 5400
   aggagagcgc acgagggagc ttccaggggg aaacgcctgg tatctttata gtcctgtcgg 5460
   gtttcgccac ctctgacttg agcgtcgatt tttgtgatgc tcgtcagggg ggcggagcct 5520
   atggaaaaac gccagcaacg cggccttttt acggttcctg gccttttgct ggccttttgc 5580
   tcacatg                                                           5587
<210> 2
   <211> 5563
   <212> DNA
   <213> Artificial
<223> DNA sequence of plasmid pVLMB10β
<400> 2
   acccgacacc atcgaatggc gcaaaacctt tcgcggtatg gcatgatagc gcccggaaga     60
   gagtcaattc agggtggtga atgtgaaacc agtaacgtta tacgatgtcg cagagtatgc    120
   cggtgtctct tatcagaccg tttcccgcgt ggtgaaccag gccagccacg tttctgcgaa    180
   aacgcgggaa aaagtggaag cggcgatggc ggagctgaat tacattccca accgcgtggc    240
   acaacaactg gcgggcaaac agtcgttgct gattggcgtt gccacctcca gtctggccct    300
   gcacgcgccg tcgcaaattg tcgcggcgat taaatctcgc gccgatcaac tgggtgccag    360
   cgtggtggtg tcgatggtag aacgaagcgg cgtcgaagcc tgtaaagcgg cggtgcacaa    420
   tcttctcgcg caacgcgtca gtgggctgat cattaactat ccgctggatg accaggatgc    480
   cattgctgtg gaagctgcct gcactaatgt tccggcgtta tttcttgatg tctctgacca    540
   gacacccatc aacagtatta ttttctccca tgaagacggt acgcgactgg gcgtggagca    600
   tctggtcgca ttgggtcacc agcaaatcgc gctgttagcg ggcccattaa gttctgtctc    660
   ggcgcgtctg cgtctggctg gctggcataa atatctcact cgcaatcaaa ttcagccgat    720
   agcggaacgg gaaggcgact ggagtgccat gtccggtttt caacaaacca tgcaaatgct    780
   gaatgagggc atcgttccca ctgcgatgct ggttgccaac gatcagatgg cgctgggcgc    840
   aatgcgcgcc attaccgagt ccgggctgcg cgttggtgcg gacatctcgg tagtgggata    900
   cgacgatacc gaagacagct catgttatat cccgccgtta accaccatca aacaggattt    960
   tcgcctgctg gggcaaacca gcgtggaccg cttgctgcaa ctctctcagg gccaggcggt   1020
   gaagggcaat cagctgttgc ccgtctcact ggtgaaaaga aaaaccaccc tggcgcccaa   1080
   tacgcaaacc gcctctcccc gcgcgttggc cgattcatta atgcagctgg cacgacaggt   1140
   ttcccgactg gaaagcgggc agtgagcggt acccgataaa agcggcttcc tgacaggagg   1200
   ccgttttgtt ttgcagccca cctcaacgca attaatgtga gttagctcac tcattaggca   1260
   ccccaggctt tacactttat gcttccggct cgtatgttgt gtggaattgt gagcggataa   1320
   caatttcaca caggaaacag ctatgaccat gattacgaat ttctagagga gccttttttt   1380
   tggagatttt caacgtgaaa aaattattat tcgcaattcc tttagttgtt cctttctatt   1440
   ctcacagtgc acttgaaacg acactcacgc agtctccact ctccctgtcc gtcacccctg   1500
   gagagtcggc ctccatctcc tgcaggtata gtcagagcct cttccacagg aattggaaaa   1560
   cctgggtgga ttggtacctg cagaagccag ggcagtctcc acaagtcctg atctatgcgg   1620
   cttctattcg ggcctccggc gtccctgaca ggttcagtgg cagtgcttca ggcacagatt   1680
   ttacactgaa aatcagcagg gtggaggctg aggatgttgg ggtttattac tgcatgcaag   1740
   gtacacaccc gtacactttt ggccagggga ccaagctgac cgtcctaggt gcggccgcgg   1800
   cgtcgggggc cgaattcgtc gacggtgcgc cggtgccgta tccggatccg ctggaaccga   1860
   tcgacaattc agccgcaatt agtatggcaa atccacgtcc accaacaccg cgggtcgctg   1920
   cggccgtatt ttcattggat gattatgatg caaaagacaa tagtgaatca tcaataggta   1980
   atttagctcg tgtaatacct agaatgggaa gggagttaat taatgattat gaagaaatcc   2040
   ccttggagga gttggaagat gaagcggaag aagaacgtcg ccaagcaacg caattccact   2100
   ccaaaagtcg taaccgtaga gctatatcat cggaaccatc atctgatgaa gatgcatctg   2160
   aatcggtttc cacatcagac aaacaccctc aagataatac ggaacttcat gaaaaagttg   2220
   agacggcggg tttacaacca agagccgcgc agccgcgaac ccaagccgcc gcgcaagccg   2280
   atgcagtcag caccaatact aactcggctt tatctgacgc aatggcaagc acgcaatcta   2340
   tcttgttgga tacaggtgct tacttaacac ggcacattgc acaaaaatca cgcgctgatg   2400
   ccgaaaaaaa cagtgtttgg atgtcaaaca ccggttatgg ccgtgattat gcttccgcac   2460
   aatatcgccg gtttagttcg aaacgcacgc aaacacaaat cggcattgac cgcagcttgt   2520
   ccgaaaatat gcagataggc ggagtattga cttactctga cagtcagcat acttttgatc   2580
   aggcgggcgg caaaaatact tttgtgcaag ccaaccttta tggtaagtat tatttaaatg   2640
   atgcttggta tgtggccggc gatattggtg cgggcagctt gagaagccgg ttacaaacgc   2700
   agcaaaaagc aaactttaac cgaacaagca tccaaaccgg ccttactttg ggcaatacgc   2760
   tgaaaatcaa tcaattcgag attgtcccta gtgcgggtat ccgttacagc cgcctgtcat   2820
   ctgcagatta caagttgggt gacgacagtg ttaaagtaag ttctatggca gtgaaaacac   2880
   taacggccgg actggatttt gcttatcggt ttaaagtcgg caaccttacc gtaaaaccct   2940
   tgttatctgc agcttacttt gccaattatg gcaaaggcgg cgtgaatgtg ggcggtaaat   3000
   ccttcgccta taaagcagat aatcaacagc aatattcagc aggcgtcgcg ttactgtacc   3060
   gtaatgttac attaaacgta aatggcagta ttacaaaagg aaaacaattg gaaaaacaaa   3120
   aatccggaca aattaaaata cagattcgtt tctaaaatac caaattcata gcaaaataaa   3180
   atgccgtctg aactcaagct tgacctgtga agtgaaaaat ggcgcacatt gtgcgacatt   3240
   ttttttgtct gccgtttacc gctactgcgt cacggatccc cacgcgccct gtagcggcgc   3300
   attaagcgcg gcgggtgtgg tggttacgcg cagcgtgacc gctacacttg ccagcgccct   3360
   agcgcccgct cctttcgctt tcttcccttc ctttctcgcc acgttcgccg gctttccccg   3420
   tcaagctcta aatcggggca tccctttagg gttccgattt agtgctttac ggcacctcga   3480
   ccccaaaaaa cttgattagg gtgatggttc acgtagtggg ccatcgccct gatagacggt   3540
   ttttcgccct ttgacgttgg agtccacgtt ctttaatagt ggactcttgt tccaaactgg   3600
   aacaacactc aaccctatct cggtctattc ttttgattta taagggattt tgccgatttc   3660
   ggcctattgg ttaaaaaatg agctgattta acaaaaattt aacgcgaatt ttaacaaaat   3720
   attaacgttt acaatttcag gtggcacttt tcggggaaat gtgcgcggaa cccctatttg   3780
   tttatttttc taaatacatt caaatatgta tccgctcatg tcgagacgtt gggtgaggtt   3840
   ccaactttca ccataatgaa ataagatcac taccgggcgt attttttgag ttatcgagat   3900
   tttcaggagc taaggaagct aaaatggaga aaaaaatcac tggatatacc accgttgata   3960
   tatcccaatg gcatcgtaaa gaacattttg aggcatttca gtcagttgct caatgtacct   4020
   ataaccagac cgttcagctg gatattacgg cctttttaaa gaccgtaaag aaaaataagc   4080
   acaagtttta tccggccttt attcacattc ttgcccgcct gatgaatgct catccggagt   4140
   tccgtatggc aatgaaagac ggtgagctgg tgatatggga tagtgttcac ccttgttaca   4200
   ccgttttcca tgagcaaact gaaacgtttt catcgctctg gagtgaatac cacgacgatt   4260
   tccggcagtt tctacacata tattcgcaag atgtggcgtg ttacggtgaa aacctggcct   4320
   atttccctaa agggtttatt gagaatatgt ttttcgtctc agccaatccc tgggtgagtt   4380
   tcaccagttt tgatttaaac gtggccaata tggacaactt cttcgccccc gttttcacca   4440
   tgggcaaata ttatacgcaa ggcgacaagg tgctgatgcc gctggcgatt caggttcatc   4500
   atgccgtctg tgatggcttc catgtcggca gaatgcttaa tgaattacaa cagtactgcg   4560
   atgagtggca gggcggggcg taattttttt aaggcagtta ttggtgccct taaacgcctg   4620
   gtgctacgcc tgaataagtg ataataagcg gatgaatggc agaaattcga aagcaaattc   4680
   gacccggtcg tcggttcagg gcagggtcgt taaatagccg cttatgtcta ttgctggttt   4740
   accggtttat tgactaccgg aagcagtgtg accgtgtgct tctcaaatgc ctgaggccag   4800
   tttgctcagg ctctccccgt ggaggtaata attgctcgac atgaccaaaa tcccttaacg   4860
   tgagttttcg ttccactgag cgtcagaccc cgtagaaaag atcaaaggat cttcttgaga   4920
   tccttttttt ctgcgcgtaa tctgctgctt gcaaacaaaa aaaccaccgc taccagcggt   4980
   ggtttgtttg ccggatcaag agctaccaac tctttttccg aaggtaactg gcttcagcag   5040
   agcgcagata ccaaatactg tccttctagt gtagccgtag ttaggccacc acttcaagaa   5100
   ctctgtagca ccgcctacat acctcgctct gctaatcctg ttaccagtgg ctgctgccag   5160
   tggcgataag tcgtgtctta ccgggttgga ctcaagacga tagttaccgg ataaggcgca   5220
   gcggtcgggc tgaacggggg gttcgtgcac acagcccagc ttggagcgaa cgacctacac   5280
   cgaactgaga tacctacagc gtgagctatg agaaagcgcc acgcttcccg aagggagaaa   5340
   ggcggacagg tatccggtaa gcggcagggt cggaacagga gagcgcacga gggagcttcc   5400
   agggggaaac gcctggtatc tttatagtcc tgtcgggttt cgccacctct gacttgagcg   5460
   tcgatttttg tgatgctcgt caggggggcg gagcctatgg aaaaacgcca gcaacgcggc   5520
   ctttttacgg ttcctggcct tttgctggcc ttttgctcac atg                     5563
<210> 3
   <211> 47
   <212> DNA
   <213> Artificial
<223> Primer VHHA1
<400> 3
   ctatgcggcc cagccggcca tggctcaggt gcagctggtg gagtctt 47
<210> 4
   <211> 21
   <212> DNA
   <213> Artificial
<223> Primer GEN III-Rev
<400> 4
   accctcatag ttagcgtaac g 21
<210> 5
   <211> 44
   <212> DNA
   <213> Artificial
<223> Primer Linker-A48-VamyA
<400> 5
   ggcggtccga ctgctaactc tggacaggtg cagctggtgg agtc 44
<210> 6
   <211> 30
   <212> DNA
   <213> Artificial
<223> Primer Vamy-Not
   <400> 6
   gagtcattct gcggccgctg aggagacggt 30
<210> 7
   <211> 60
   <212> DNA
   <213> Artificial
<223> Primer Linker-A48
<400> 7
   accccgtctc acaactccca ccaggttcca tccgcaggcg gtccgactgc taactctgga 60
<210> 8
   <211> 37
   <212> DNA
   <213> Artificial
<223> Primer Linker -A48-Vamy-eag1
<400> 8
   attactcgcc ggccggtacc ccgtctcaca actccca 37
<210> 9
   <211> 33
   <212> DNA
   <213> Artificial
<223> Primer VL1
<400> 9
   gagtcattct agaggagcct tttttttgga gat 33
<210> 10
   <211> 26
   <212> DNA
   <213> Artificial
<223>. Primer VL2
<400> 10
   ctgagatgag tttttgttct gcggcc 26